# EUROPEAN PATENT APPLICATION

(11) **EP 2 008 670 A1**
(43) Date of publication of application: **31.12.2008**
(21) Application number: 07737917.0
(22) Date of filing: 07.03.2007
(51) Int. Cl.: A61L 2/14, B65B 55/04, B65B 55/10, H05H 1/24

(54) **STERILIZER AND STERILIZATION METHOD USING THE SAME**

(30) Priority: 07.03.2006 JP 2006061674; 18.02.2007 JP 2007037254
(71) Applicant: University of The Ryukyus, Nakagami-gun, Okinawa 903-0213 (JP); SAGA UNIVERSITY, Saga-Shi Saga 840-8502 (JP); The Coca-Cola Company, Atlanta, GA 30309-3996 (US)
(72) Inventor: YONESU, Akira, University of the Ryukyus, Nakagami-gun, Okinawa 9030213 (JP); HAYASHI, Nobuya, Saga University, Saga-shi, Saga 8408502 (JP); TACHIBANA, Yoshihisa, Shibuya-ku, Tokyo 1500002 (JP)
(74) Representative: Staudt, Hans-Peter
(86) International application number: PCT/JP2007/054403
(87) International publication number: WO 2007/102532

(57) **Abstract**

It is intended to provide a sterilizer, which aims at efficiently sterilizing a subject such as a container by using active oxygen generated with the use of plasma, and a sterilization method using the same. In particular, it is intended to develop a technique for stably generating active oxygen with the use of plasma and thus provide a sterilizer , which enables, if necessary, stable plasma formation and active oxygen generation in the atmosphere and can inhibit thermal damages caused by the plasma on a subject to be sterilized, and a sterilization method using the same. A sterilizer wherein a gas (100), which contains at least one of active oxygen generated by converting oxygen gas into plasma and active oxygen generated by converting a gas other than oxygen into plasma and contacting the plasma with oxygen gas, is brought into contact with a subject (101) to thereby kill microorganism sticking to the subject, **characterized in that** a non-conductive gas channel tube (1) for introducing the gas to be converted into plasma and discharging into the atmosphere and a conductive antenna tube (2) located around the gas channel tube are provided, the antenna tube has a slit (3) in a definite length formed along the tube axis direction of the gas channel tube, and the antenna tube is irradiated with microwave to thereby convert the gas in the gas channel tube into plasma. In a preferable mode, the above sterilizer is **characterized in that** the slit has a length corresponding to an integral multiple of the half-wave length of the microwave to be used in the irradiation.

## Description

### Technical Field

This invention relates to a sterilizer and a sterilizing method using the same, and in particular, to a sterilizer for putting a gas which includes at least active oxygen generated by converting an oxygen gas to plasma or active oxygen generated by converting a gas other than oxygen to plasma and putting the plasma in contact with an oxygen gas in contact with an object, so that bacteria which adhere to the object can be killed, and a sterilizing method using the same.

### Background Art

Though in accordance with conventional methods for sterilizing containers and medical instruments, ultraviolet rays and high pressure steam gas are used, methods using hydrogen peroxide converted to plasma in a Sterilizing process have also been proposed, in order to improve the sterilizing effects.

Sterilizing processes using plasma can be roughly categorized into methods using the high temperature properties of plasma and methods using a gas in a temporarily active state when the gas that is converted to plasma returns to a conventional gas.
Patent Document 1 discloses a method for generating plasma through are discharge by applying a high frequency voltage across electrodes, specifically, a method for heating and molding the tip of syringe needles using plasma and at the same time carrying a sterilizing process.
Patent. Document 1: Japanese Unexamined Patent Publication H6 (1994)-197930

One problem caused by arc discharged is that electrons and ions generated between the electrodes collide with the electrodes, making the electrode of a high temperature, and thus, electrodes wear out, and in addition, part of the metal material that forms the electrodes is released into the plasma, and thus, there is a possibility that impurities may mix into the plasma. Another problem is that when an object is sterilized with plasma at a high temperature, the surface of the object two be sterilized may change in equality.

Meanwhile, in accordance with the method using hydrogen peroxide plasma disclosed in Patent Document 2, hydrogen peroxide, together with the object to be sterilized, is put in a bag which transmits microwaves, and the hydrogen peroxide is converted to plasma through irradiation with microwaves, so that active oxygen is generated and the object is sterilized.
One problem is that hydrogen peroxide is toxic if the solution used is of a high concentration (50 % or higher) is used, and in addition, clothing may dissolve, and thus caution is required on the part of the user. Another problem is that in order to prevent hydrogen peroxide gas from spreading, it is necessary to carry out the sterilizing process within a closed container, and thus, continuous processing becomes difficult.
Patent Document 2: Japanese Unexamined Patent Publication 2005-279042

Patent Document 3, by Saga University, which is one of the present applicants, discloses a sterilizing process in which an oxygen gas is converted to plasma without using hydrogen peroxide and oxygen radicals are generated. Here, in Patent Document 3, oxygen radicals are generated with high density, and therefore, the inside of the sterilizer is kept in a low pressure state.
Patent Document 3: Japanese Unexamined Patent Publication 2006-20950

In accordance with methods using electrons and ions in plasma for sterilization, the life of electrons and ions is in the microseconds-hence very short-and the range is in the millimeters or less, and therefore, the finer parts of complicated structures (bottle caps and the like) cannot be sterilized. Oxygen atoms gained through oxygen discharge: so-called oxygen radicals as those in Patent Document 3, however, have a life of several tens of milliseconds and a range of several centimeters, and therefore permeate into the finer parts of containers having complicated structures, thus making sterilization possible. Here, oxygen radicals are a type of active oxygen.
In order to sterilize a large amount of containers for potable water and foods with active oxygen generated using plasma as that described above, a technology for generating plasma stably under ambient pressure is indispensable, and in addition, it becomes necessary to make low temperature processing at 70 °C or less possible, in order to carry out a sterilizing process on PET containers and the like.

### Disclosure of the Invention

### Problem to Be Solved by the Invention

An object of the present, invention is to solve the above described problems and provide a sterilizer for efficiently carrying out a sterilizing process on such objects as containers using active oxygen generated using plasma, as well as a sterilizing method using the same. In particular, an object of the invention is to provide a technology for generating active oxygen stably using plasma, and a sterilizer which makes generation of plasma and active oxygen which are stable under ambient pressure possible if necessary, and can prevent plasma from thermally damaging the object to be sterilized, as well as a sterilizing method using the same.

### Means for Solving Problem

The invention according to Claim 1 provides a sterilizer for putting a gas which includes at least, active oxygen generated by converting an oxygen gas to plasma our active oxygen generated by converting a gas other than oxygen to plasma and putting the plasma in contact with an oxygen gas in contact with an object so that bacteria which adhere to the object can be killed, characterized in that the sterilizer has: a non-conductive gas flow pipe through which a gas to be converted to plasma is introduced and discharged into the air; and a conductive antenna pipe surrounding the gas flow pipe, wherein a slit of a predetermined length is created in the antenna pipe along the pipe axis of the gas flow pipe, and the antenna pipe is irradiated with microwaves to that the gas flowing through the gas flow pipe is converted to plasma.

"Active oxygen" as used in the present invention is active oxygen generated by converting an oxygen gas to plasma or active oxygen generated by converting a gas other than oxygen to plasma and putting the plasma in contact with an oxygen gays, and refers to oxygen atoms or molecules, or molecules including oxygen which are in a more active state than conventional oxygen molecules (O₂).

The invention according to Claim 2 provides the sterilizer according to Claim 1, **characterized in that** the slit is open on the side of the gas flow pipe from which the gas is discharged.

The invention according to Claim 3 provides the sterilizer according to Claim 1, **characterized in that** the slit is created inside the antenna pipe.

The invention according to Claim 4 provides the sterilizer according to any of Claims 1 to 3, **characterized in that** the end portion of the antenna pipe on the side from which the gas is discharged from the gas flow pipe is bent toward the gas flow pipe.

The invention according to Claim 5 provides the sterilizer according to any of Claims 1 to 4, **characterised in that** the length of the slit is set to a multiple of the half wavelength of microwaves for irradiation in integers.

The invention according to Claim 6 provides the sterilizer according to any of Claims 1 to 5, **characterized in that** the gas flow pipe protrudes from the end portion of the antenna pipe on the downstream side in the direction of the gas flow, and the length of the protruding portion is greater than the length of the plasma torch.

The invention according to Claim 7 provides the sterilizer according to any of Claims 1 to 6, **characterized in that** the gas flow pipe and the antenna pipe can be moved relative to each other in the configuration.

The invention according to Claim 8 provides the sterilizer according to any of Claims 1 to 7, **characterized in that** an oxygen gas pipe for introducing an oxygen gas is provided inside the gas flow pipe or around the gas flow pipe, so that plasma generated inside the gas flow pipe and the oxygen gas introduced through the oxygen gas pipe make contact.

The invention according to Claim 9 provides the sterilizer according to any of Claims 1 to 8, **characterized in that** the object of sterilization is a container or a lid of a container.

The invention according to Claim 10 provides the sterilizer according to Claim 9, characterized by comprising a moving/rotating means for moving or rotating the object relative to the gas flow pipe.

The invention according to Claim 11 provides a sterilizing method using the sterilizer according to Claim 10, **characterized in that** the object of sterilization is a container, comprising the insertion step of inserting an end of the gas flow pipe so that the end reaches the vicinity of the deepest portion inside the container; the filling step of filling the container with the gas discharged from the gas flow pipe in the state after the insertion step; and the drawling step of drawing the gas flow pipe from the vicinity of the deepest portion inside the container to in the vicinity of the opening of the container after the filling step.

The invention according to Claim 12 provides the sterilizing method according to Claim 11, **characterized in that** the insertion step and the drawing step are carried out by moving the gas flow pipe and the container relative to each other along the pipe axis of the gas flow pipe.

The invention according to Claim 13 provides the sterilizing method according to Claim 12, **characterized in that** the speed of the relative movement, between the gas flow pipe and the container is slower in the drawing step than in the insertion step.

The invention according to Claim 14 provides a sterilizing method using the sterilizer according to any of Claims 1 to 8, **characterized in that** the object of sterilization is a container, the container contains part of the gas flow pipe and the antenna pipe, and a shielding means is provided so as to surround the container, and thus, the antenna pipe can be irradiated with microwaves while the gas is being discharged into the container from the gas flow pipe.

The invention according to Claim 15 provides the sterilizing method according to Claim 14, **characterized in that** a discharge hole for discharging the gas from inside the shielding means is created in a portion of the shielding means, so that a gas can be supplied to the gas flow pipe while air is discharged through the discharge hole.

The invention according to Claim 16 provides a sterilizing method using the sterilizer according to any of Claims 1 to 8, **characterized in that** the object of sterilization is a container, and a space for temporarily storing the gas discharged from the gas flow pipe is provided, so that the container can be conveyed through the space.

The invention according to Claim 17 provides the sterilizing method according to Claim 16, characterized in that the space is a tunnel, and the discharge end of the gas flow pipe connects to the space.

The invention according to Claim 18 provides a sterilizing method using the sterilizer according to any of Claims 1 to 8, **characterized in that** the object, of sterilization is a container, a gas discharged from the gas flow pipe is blown against the outer surface of the container, and the container is rotated around the center axis in a direction approximately perpendicular to the pipe axis of the gas flow pipe.

The invention according to Claim 19 provides the sterilizing method according to Claim 18, **characterized in that** a hood for putting the gas discharged from the gas flow pipe in contact with the container is provided in the vicinity of the discharge end of the gas flow pipe.

The invention according to Claim 20 provides a Sterilizing method using the sterilizer according to any of Claims 1 to 8, **characterized in that** the object of sterilization is a lid of a container, the discharge end of the gas flow pipe is directed toward the inside of the lid so that a gas can be supplied to the lid from the gas flow pipe, and the lid can be rotated.

The invention according to Claim 21 provides the sterilizing method according to Claim 20, **characterized in that** the speed of rotation and the direction of rotation of the lid is changed over time.

The invention according to Claim 22 provides a sterilizing method using the sterilizer according to any of Claims 1 to 8, **characterized in that** the object of sterilization is a lid of a container, and the lid is put in a vacuum container, so that, a gas can be supplied into the vacuum container from the discharge end of the gas flow pipe while air is discharged from the vacuum container.

The invention according to Claim 23 provides a sterilizing method using the sterilizer according to any of Claims 1 to 8, characterized by the plasma igniting step of supplying a gas which is easier to convert to plasma than oxygen gas to the gas flow pipe and igniting the gas into plasma through irradiation with microwaves, and supplying an oxygen gas together with the above described gas after the plasma igniting step so that the oxygen gas is converted to plasma.

The invention according to Claim 24 provides the sterilizing method according to Claim 23, **characterized in that** the gas supplied in the plasma igniting step is an argon gas.

### Effects of the Invention

In the invention according to Claim 1, a gas to be converted to plasma is introduced into a non-conductive gas flow pipe and a conductive antenna pipe surrounding the gas flow pipe is provided and a slit of a predetermined length is created in the antenna pipe along the pipe axis of the gas flow pipe, and therefore, an electrical field for excitation generated by microwaves is concentrated in the slit portion, so that it becomes possible to convert the gas that passes through the gas flow pipe stably to plasma in the slit portion.

In the invention according to Claim 2, the slit is open on the side of the gas flow pipe from which a gas is discharged, and therefore, it becomes possible to stably generate a plasma torch which extends outward from the end of the antenna pipe on the side of the gas flow pipe from which a gas is discharged.

In the invention according to Claim 3, the slit is created inside the antenna pipe, and therefore, it becomes possible to stably generate plasma inside the antenna pipe.

In the invention according to Claim 4, the end portion of the antenna pipe on the side from which a gas is discharged from the gas flow pipe is bent toward the gas flow pipe, and therefore, it becomes possible to stably generate plasma inside the antenna pipe.

In the invention according to Claim 5, the length of the above described slit is set to a multiple of the half wavelength of the microwaves for irradiation in integers, and therefore, a stable standing wave can be formed in the slit portion, so that the electrical field for excitation is efficiently concentrated, and thus, it becomes possible to generate plasma stably.

In the invention according to Claim 6, the above described gas flow pipe protrudes from the end portion of the antenna pipe on the downstream side of the gas flow and the length of the protruding portion is greater than the length of the plasma torch, and therefore, the object can be prevented from making contact with the plasma torch, and thus, it becomes possible to efficiently supply only active oxygen generated by the plasma to the objects,

In the invention according to Claim 7, the above described gas flow pipe and the above described antenna pipe can be moved relative to each other in the configuration, and therefore, it becomes possible to move the end portion of the gas flow pipe to an optimal location in each step; at the time of plasma ignition, at the time of the sterilizing process, and during the sterilizing process.

In the invention according to Claim 8, an oxygen gas pipe for introducing an. oxygen gas is provided inside the gas flow pipe or around the gas flow pipe, and the plasma generated inside the gas flow pipe and an oxygen gas introduced through the oxygen gas pipe make contact, and thus, it becomes possible to stably generate active oxygen using the plasma generated in the gas flow pipe. In particular, the plasma generating portion and the active oxygen generating portion can be separated, and thus, it becomes possible to use a gas which is easy to convert to plasma in the plasma generating portion.

In the invention according to Claim 9, the object of sterilization is a container or the lid of a container, and therefore, it becomes possible to carry out a sterilizing process on the object using active oxygen.

In the invention according to Claim 10, a moving/rotating means for moving or rotating the object relative to the above described gas flow pipe is provided, and therefore, it becomes possible to carry out a sterilizing process on all of the surfaces of the object, and at the same time, it becomes possible for active oxygen to efficiently make contact with these surfaces.

In the invention according to Claim 11, the object of sterilization is a container, and the insertion step of inserting an end of the gas flow pipe so that the end reaches the vicinity of the deepest portion inside the container; the filling step of filling the container with the gas discharged from the gas flow pipe in the state after the insertion step; and the drawing step of drawing the gas flow pipe from the vicinity of the deepest portion inside the container to in the vicinity of the opening of the container after the filling step are provided, and therefore, it becomes possible to replace the air within the container with active oxygen, and at the same time stably carry out a sterilizing process on the inner surface of the container.

In the invention according to Claim 12, the above described insertion step and the above described drawing step are carried out by moving the gas flow pipe and the container relative to each other along the pipe axis of the gas flow pipe, and therefore, it becomes possible to supply active oxygen to necessary portions inside the container.

In the invention according to Claim 13, the speed of the relative movement between, the gas flow pipe and the container is slower in the above described drawing step than in the above described insertion step, and therefore, the air inside the container can be efficiently replaced with active oxygen in the insertion step, and it becomes possible to supply active oxygen appropriately along the inner surface of the container in the drawing step.

In the invention according to Claim 14, the object of sterilization is a container, the container contains part of the gas flow pipe and the antenna pipe, and a shielding means is provided so as to surround the container so that the antenna pipe can be irradiated with microwaves, while a gas is discharged into the container from the gas flow pipe, and therefore, the end of the antenna pipe can be contained inside the container even in the case where the container is deep, and it becomes possible to stably supply active oxygen in the deepest portion.

In the invention according to Claim 15, a discharge hole for discharging air from inside the shielding means is created in a portion of the above described shielding means, and a gas is supplied to the gas flow pipe while air is being discharged through the discharge hole, and therefore, it becomes possible to replace the air within the container with active oxygen more rapidly.

In the invention according to Claim 16, the object of sterilization is a container, and a space for temporarily storing the gas discharged from the gas flow pipe is created, so that the container can be conveyed through the space, and therefore, it becomes possible to continuously sterilize the container, specifically, to carry out a sterilizing process on the surface of the container simply by allowing the container to pass through the space filled with active oxygen.

In the invention according to Claim 17, the above described space is a tunnel, and the discharge end of the gas flow pipe is connected to the space, and therefore, the container can be efficiently sterilized by the active oxygen with which the space is filled when the container passes through the tunnel space. In addition, air is pushed out from the space as the container passes through, and at the same time, new active oxygen keeps being supplied from the gas flow pipe, and therefore, it becomes possible to always hold fresh active oxygen inside the space.

In the invention according to Claim 18, the object of sterilization is a container, the gas discharged from the gas flow pipe is blown against the outer surface of the container, and at the same time, the container is rotated around the center axis , which is approximately perpendicular to the pipe axis of the gas flow pipe, and therefore, it becomes possible to carry out a sterilizing process on the entirety of the outer surface of the container.

In the invention according to Claim 19, a hood for putting the gas discharged form the above described gas flow pipe in contact with the container is provided in the vicinity of the discharge end of the gas flow pipe, and therefore, it become possible to efficiently supply the gas discharged from the gas flow pipe to the outer surface of the container.

In the invention according to Claim 20, the objects, of sterilization is the lid of a container, the discharge end of the gas flow pipe is directed toward the inside of the lid, a gas is supplied to the lid from the gas flow pipe, and at the same time, the lid is rotated, and therefore, it becomes possible to supply active oxygen to the inner surface of the lid while air held in the space inside the lid is discharged.

In the invention according to Claim 21, the speed of rotation and direction or rotation of the above described lid are changed over time, and therefore, it becomes possible to change the air pressure inside the lid and efficiently send active oxygen inside using the change in the air pressure.

In the invention according to Claim 22, the object of sterilization is the lid of a container, the lid is put in a vacuum containers, and a gas is supplied into the vacuum container through the discharge end of the gas flow pipe while air is discharged from the vacuum container, and therefore, it becomes possible to keep the air tat remains inside the lid to the minimum through a vacuum process, supply active oxygen to the inside of the lid, and always keep supplying new active oxygen to the lid.

In the invention according to Claim 23, the plasma igniting step of supplying a gas which is easier to convert to plasma than oxygen gas to the gas flow pipe and igniting the gas into plasma through irradiation with microwaves is provided, and an oxygen gas is supplied together with said gas after the plasma igniting step so that the oxygen gas is converted to plasma, and therefore, it becomes possible to improve the ignition of plasma for oxygen gas, which is difficult to convert to plasma.

In the invention according to Claim 24, the gas supplied in the plasma igniting step is an argon gas, and therefore, plasma can be easily ignited, and after that the state of plasma maintained when an oxygen gas is supplied, and furthermore, it becomes possible to aid the conversion of oxygen gas to plasma.

### Brief Description of the Drawings

Fig 1 is a diagram schematically showing a plasma generating portion used in the present invention;
Fig 2 is a diagram schematically showing a plasma generator used in the present invention;
Fig 3 is a diagram showing a case where the plasma generator used in the present invention has a number of plasma generating portions;
Fig 4 is a diagram showing a case where a number of plasma generating portions are driven using a single microwave generator in the plasma generator used in the present invention;
Fig 5 is a diagram showing a case where are discharge is used as an auxiliary igniting means in a plasma generating portion;
Fig 6 is a diagram showing a case where a sub-antenna pipe is used as an auxiliary igniting means in a plasma generating portion;
Fig 7 is a diagram showing a method for improving the ignition of plasma using a number of gases;
Fig 8 is a graph showing a method for introducing microwaves through pulse drive when plasma is generated;
Fig 9 is a diagram showing the sterilizing method according to the present invention;
Fig 10 is a diagram illustrating a method for sterilizing the inside of a container;
Fig 11 is a diagram illustrating a sterilizing method in the case where a container is contained within a shielding means;
Fig 12 is a diagram showing an example of a method for sterilizing the outside of a container;
Fig 13 is a diagram showing another example of a method for sterilizing the outside of a container;
Fig 14 is a diagram illustrating a method for sterilizing the outside of a container using a tunnel in cylindrical form;
Fig 15 is a diagram showing a method for sterilizing the lid of a container;
Fig 16 is a diagram showing a method for sterilizing the lid of a container using a vacuum container;
Fig 17 is a diagram schematically showing a plasma generating portion where a slit is created inside the antenna pipe;
Fig 18 is a diagram schematically showing a plasma generating portion in the case where the end portion of the antenna pipe is bent;
Fig 19 is a diagram showing a method for supplying an oxygen gas to generated plasma;
Fig 20 is a schematic diagram showing the sterilize (plasma generator) used in Example 2;
Fig 21 is a graph showing the distribution in the light emission spectrum when plasma is generated;
Fig 22 is a graph showing the intensity of light emitted from the plasma against the oxygen gas content in an argon gas;
Fig 23 is a graph showing the intensity of light emitted from the plasma against the power of the inputted microwaves;
Fig 24 is a graph showing the distribution in the temperature of the gas against the oxygen gas content; and
Fig 25 is a graph showing the distribution in the temperature of the gas against the flow rate of the gas.

### Explanation of Symbols

- 1,201, 301: gas flow pipe
- 2, 202, 302: antenna pipe
- 2': sub-antenna pipe
- 3, 203, 303: slit
- 4, 204: plasma torch
- 5,40, 110: shielding means
- 6: microwave generator
- 7, 11, 12, 13, 14, 61, 62, 63, 64: microwaves
- 8, 70, 71: gas source
- 9, 74, 306: gas
- 10: microwave intensity adjusting means
- 50: electrode for arc discharge
- 51: high voltage power supply
- 72, 73: valve
- 80: pulse waveform of power for emitting microwaves
- 100: gas including active oxygen
- 101: object
- 102: container
- 111, 163: discharged gas
- 120, 152: roller
- 121: hood
- 130, 140: tunnel
- 131, 132: direction in which container conveyed
- 141: guiding means
- 150, 161: lid of container
- 151: sealing member
- 160: vacuum container
- 162: shelf
- 205: bent portion
- 305: oxygen gas pipe
- 307: oxygen gas
- 308: plasma gas

### Best Mode for Carrying Out the Invention

The sterilize and sterilizing method using the same according to the present invention are described in detail below.

### (Plasma generating Portion)

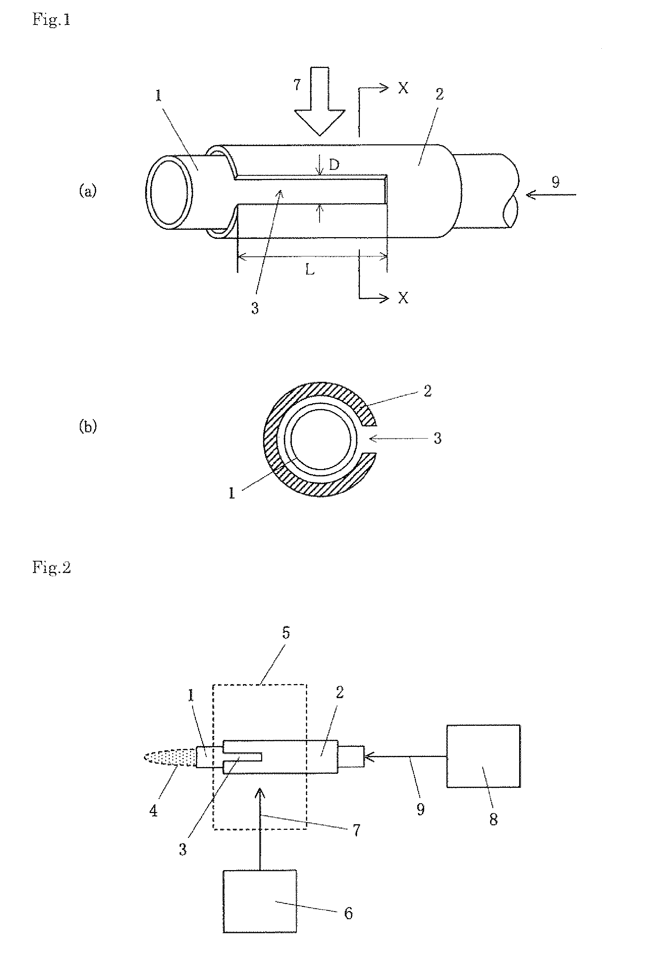

Fig 1(a) shows the structure of a plasma generating portion used in a sterilizer. The plasma generating portion is formed of a non-conductive gas flow pipe 1, for example a crystal pipe, and a conductive antenna pipe 2, for example an aluminum pipe, and the conductive antenna pipe 2 is provided so as to surround the gas flow pipe 1.

The plasma generating portion used in the present invention is characterized in that a slit 3 is created in the conductive antenna pipe 2. An electrical field for excitation generated by microwaves with which the plasma generating portion is irradiated is concentrated in this slit portion, and thus, it becomes possible for plasma to be generated and remain in the gas flow pipe, as a result of this electrical field.
As for the form of the slit 3, the length L of the slit portion is set to a multiple of the half wavelength of the wavelength λ of the microwaves with which the plasma generating portion is irradiated in integers (nλ/2; n is an integer of one or greater). In addition, though the width D of the slit portion is not particularly limited, the intensity of the electrical field for excitation generated in the slit portion increases as the width D becomes smaller, thus making it possible to accelerate conversion of the gas that passes through the gas flow pipe to plasma while the area around the gas flow pipe where an electrical field for excitation is generated becomes smaller, and therefore, the amount of gas that can be converted to plasma is reduced.

The number of slits 3 provided in the antenna pipe 2 is not limited to one, as in Fig 1(a). Fig 1(b) is a cross sectional diagram along arrows X-X in Fig 1(a), where the antenna pipe 2 is concentric with the gas flow pipe 1, and the cross section of the antenna pipe 2 is in C shape, due to the slit 3, When the number of slits 3 is increased, an electrical field for excitation can be generated in each slit, and thus, it becomes possible to increase the number of places where the gas that passes through the gas flow pipe is converted to plasma.

As for the form and location of the above described slit, as shown in Fig 1(a), a slit which is open in the end portion of the antenna pipe (end portion on side from which gas is discharged from gas flow pipe) is illustrated, and in the case where such a slit is used, it becomes possible to stably form a plasma torch which extends outward from the end of the antenna pipe on the side from which a gas is discharged from the gas flow pipe.

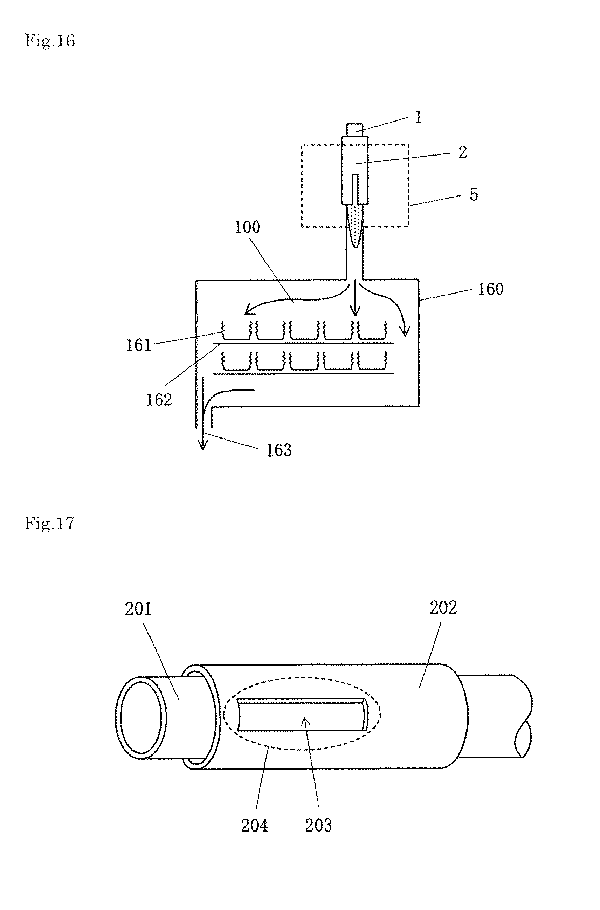

Meanwhile, in order to stably generate plasma inside the antenna pipe, as shown in Fig 17, a slit 203 is created inside the antenna pipe 202. As a result, it becomes possible to generate plasma 204 inside the glass flow pipe 201 in the vicinity of the slit 203. The plasma torch which extends outward from the antenna pipe and the gas flow pipe is appropriate for use in the case where the plasma is directly irradiated. In the case where the plasma is not directly irradiated, however, it is necessary to secure a sufficient distance between the end of the gas flow pipe and the end of the antenna pipe, as described below. It is also possible to shorten the distance by using the antenna pipe 202 in Fig 17.

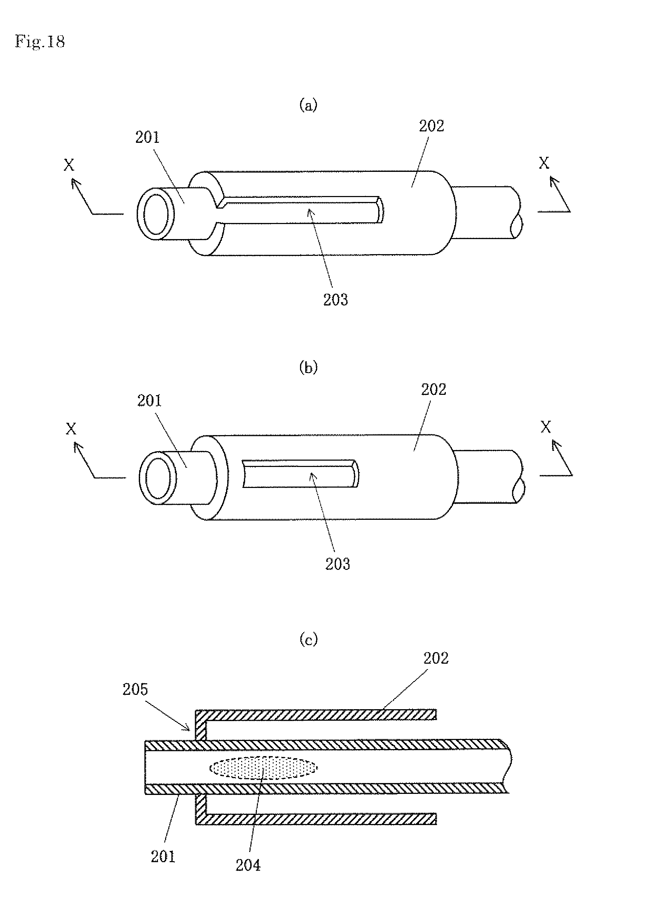

It is also possible to adopt, an antenna pipe in the form shown in Fig 18 in accordance with another method for generating plasma inside the antenna pipe. The form of the antenna pipe in Fig 18(a) is similar to that in Fig 1(a), except that the end portion of the antenna 202 pipe (end portion of gas flow pipe 201 on side from which gas discharged) is bent toward the gas flow pipe 201, as shown in Fig 18(c), which is a cross sectional diagram along arrows X-X in Fig 18(a). The plasma 204 is generated inside the antenna pipe 202 due to this bent portion 205, and a plasma torch is prevented from being generated.

Furthermore, antenna pipe shown in Fig 18(b) is similar to that in Fig 17, and a bent portion 205 is formed in the end portion of the antenna 202 pipe. The cross sectional diagram along arrows X-X in Fig 18(b) is similar to Fig 18(c).
The present inventors found out that plasma can be generated inside the antenna pipe, and in addition, plasma ignition can be improved by creating a slit inside the antenna pipe and forking a bent portion in an end portion of the antenna pipe as shown in Figs 17 and 18.
Here, though mainly an example of an antenna pipe having a slit in the form shown in Fig 1(a) is described below, the antenna pipes having a slit shown in Figs 17 and 18 can, of course, be applied in the same manner.

Next, the operation of the plasma generating portion is described.
A gas 9 to be converted to plasma is introduced into the gas flow pipe 1, and the gas keeps flowing in one direction. When the plasma generating portion is irradiated with microwaves 7 in this state, a standing wave of microwaves is created in the slit portion of the antenna pipe 2, and a concentrated electrical field for excitation is generated. The electrical field for excitation penetrates the gas flow pipe so as to heat the gas and generate plasma. The generated plasma proceeds along the gas flow in the direction of the exit of the gas flow pipe 1 (to the left in the figure), and specifically, in the case where a slit in the form shown in Fig 1(a) is used, plasma in torch form (referred to as "plasma torch") is released from the exit of the flow pipe 1.

Though the gas used in the present invention is oxygen, it is possible to mix oxygen with various types of gases, such as argon, helium and hydrogen, for use if necessary. In addition, as described below, it is also possible to first introduce a gas which is easier to convert to plasma than oxygen gas, such as argon, into the gas flow pipe, and after the ignition of plasma supply an oxygen gas in the configuration, in order to improve the ignition of plasma.

It is possible to change the characteristic of the plasma torch, for example the electron temperature, the gas temperature, the plasma density, the density of radical gases and the length of the torch (length between opening in gas flow pipe or end of antenna pipe and end of plasma torch) by adjusting the power for irradiating the plasma generating portion with microwaves and the amount of gas flow.

### (Plasma Generator)

Fig 2 is a schematic diagram showing the basic configuration of a plasma generator which includes a plasma generating portion as described above.
A predetermined amount of a gas 9 is supplied to a gas flow pipe I which forms the plasma generating portion from a gas source 8, for example a gas tank for storing a gas for generating plasma, tor example an oxygen gas. An antenna pipe 2 which surrounds the gas flow pipe I is contained in a shielding means 5 for containing microwaves, and one end of the antenna pipe 2 (end portion on side where no slit 3 created; two end sides in case where slit in antenna pipe, as in Fig 17) is electrically connected to the shielding means 5. The shielding means is a portion corresponding to the cavity in the prior art, and in the following, the term "shielding means" includes cavities.

Microwaves 7 are introduced into the shielding means 5 from a microwave generator 6, so that the antenna pipe 2 is irradiated with microwaves 7. The slit 3 in the antenna pipe 2 allows microwaves to form a standing wave, so that an electrical field for excitation is generated. The electrical field for excitation converts the gas which passes through the gas flow pipe 1 to plasma, so that a plasma torch 4 is generated and discharged from the end of the gas flow pipe 1.

Though the material and form of the shielding means 5 are not particularly limited, as long as the shielding means can contain microwaves, it is preferable to use a container made of stainless steel in order to hold the plasma generating portion within the shielding means, and efficiently reflect microwaves.
In addition, it is preferable for microwaves to be in such a form as to easily resonate, so that microwaves can be efficiently contained within the shielding means 5, and thus, it is possible to make a portion of the wall that forms the shielding means movable, so that the volume and form inside the shielding means are adjustable.

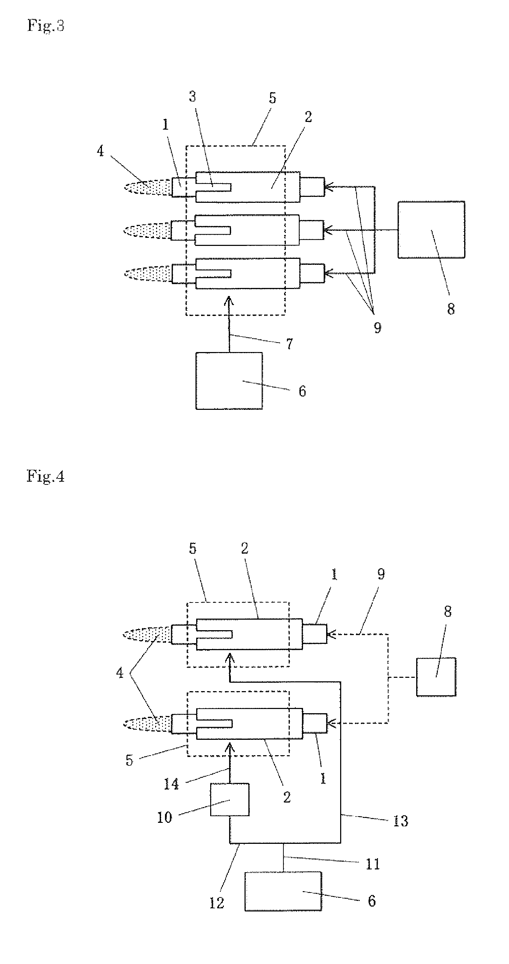

Fig 3 shown a number of plasma generating portions provided inside the shielding means 5. Plasma generation according to the present invention is characterized in that, plasma is generated by the electrical field for excitation generated in the slit 3 created in the antenna pipe 2, and therefore, it is possible for plasma to be generated and remain in the gas flow pipe appropriately even in the case where a number of plasma generating portions are provided inside the shielding means 5. Here, as shown in Fig 3, it is possible to apply a method for splitting gas supplied from the gas source 8 and supplying it into each gas flow pipe 1 forming the plasma generating portion, as well as to provide a gas source to each gas flow pipe 1.

In addition, as shown in Fig 4, it is also possible to provide separate shielding means 5 for each plasma, generating portion. This is because loss of microwaves can be better prevented and plasma generated more efficiently when separate shielding means are provided for each plasma generating portion than when a single shielding means surrounds all of the antenna pipes in the case where a number of separate plasma generating portions are provided or the antenna, pipes of the respective plasma generating portions are oriented in different directions.

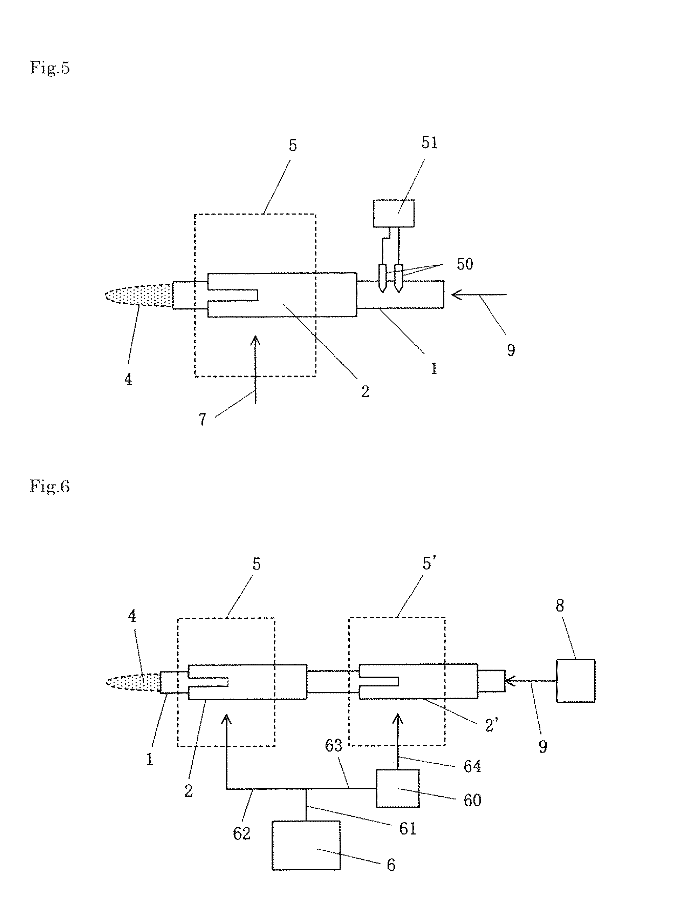

As a method for supplying microwaves into a number of shielding means 5, though it is possible to provide microwaves generators to separate shielding means, microwaves 11 from a single microwave generator 6 can be split, so that split microwaves 12 and 13 can be supplied to different shielding means 5 in the configuration, as shown in Fig 5. Here, it is also possible to provide an intensity adjusting means 10 for adjusting the intensity of microwaves in a portion of the waveguide for guiding microwaves 12 in at least one direction, in order to optimize the intensity of the microwaves supplied into the shielding means. Here, it is, of course, possible to install an isolator or a tuner between the microwave generator and the shielding means in the plasma generator according to the present invention if necessary.

### (Auxiliary Igniting Means)

At the time of plasma ignition, it is possible to improve plasma ignition by keep the pressure inside the gas flow pipe 1 (approximately 10² Pa to 10³ Pa; here, the set pressure varies in accordance with the frequency and power of microwaves, as well as the type of gas to be converted to plasma) lower than the ambient, pressure (approximately 10⁵ Pa). In this case, an air discharging pipe is connected to an end of the gas flow pipe 1, and the gas discharging pipe is removed after the plasma ignition, so that the inside of the gas flow pipe becomes of the ambient pressure.
In addition to this method for providing low pressure, it is also possible to use the arc discharge means shown in Fig 5, or an auxiliary igniting means, such as the microwave heating means shown in Fig 6, in combination. This auxiliary igniting means makes it possible to facilitate plasma ignition under the ambient pressure, thus making it unnecessary to use a discharge pipe and a vacuum pump, as in the case of low pressure, and therefore, the configuration of the plasma generator can be made simple.

In the are discharge means, two electrodes 50 as those shown in Fig 5 are provided so as to protrude inside the gas flow pipe 1, and are discharge is initiated between the two by means of a high voltage supply 51. The gas that is once discharged can be easily converted to plasma by the electrical field for excitation generated by the antenna pipe 2, and therefore, it is not necessary to keep the inside of the gas flow pipe at a pressure lower than the ambient pressure. In addition, it is not necessary for the arc discharge to be continuous discharge, and it may be discharge in pulses. Are discharge stops naturally after the ignition of plasma by means of the antenna pipe 2.

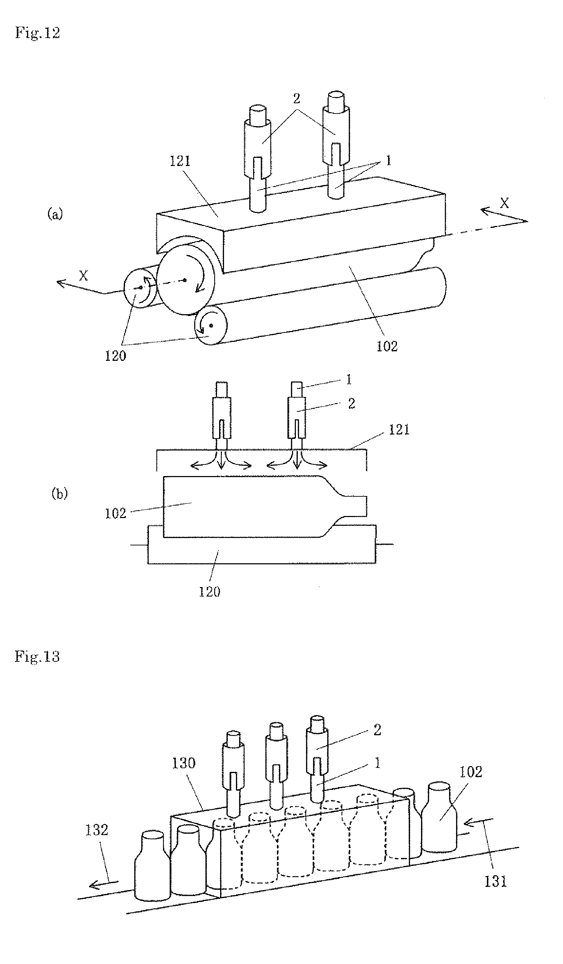

Fig 6 shows a method for providing a sub-antenna pipe 2' for auxiliary ignition on the side upstream from the gas flow pipe 1, and converting part of the gas to plasma prior to conversion of plasma by means of the main antenna pipe 2.
In addition to using separate shielding means 5 and 5' which surround the respective antenna pipes 2 and 2', as shown in Fig 12, it is also possible for the antenna pipes 2 and 2' to share a single shielding means. Here, it is preferable for separate shielding means to be provided, so that the respective antenna pipes can be irradiated with appropriate microwaves.

The sub-antenna pipe 2' may allow part of the gas that passes through the gas flow pipe to be converted to plasma, and the sub-antenna pipe 2' can be formed in such a manner that the width of the slit is smaller than that of the main antenna pipe 2 and the electrical field for excitation can be locally increased, for example. In addition, it is also possible to make the diameter of the gas flow pipe 1 smaller in the location of the sub-antenna pipe 2', and the sub-antenna pipe 2' has a diameter smaller than the main antenna pipe, and thus, it is possible to increase the electrical field for excitation.

In the case where the two antenna pipes share the microwave generator 6 for supplying microwaves, as shown in Fig 6, microwaves 61 emitted from the microwave generator 6 are split, so that the main antenna pipe 2 is irradiated with microwaves 62 in one direction. In addition, the microwaves 63 in the other direction are converted to microwaves 64 via a microwave blocking means 60, so that the sub-antenna pipe 2' is irradiated with microwaves 64 in the configuration. The microwave blocking means 60 guides microwaves 63 at the time of auxiliary ignition and blocks microwaves 63 when auxiliary ignition becomes unnecessary. In addition, it is also possible to provide an adjusting means (not shown) for adjusting the intensity of microwaves in the waveguides for split microwaves if necessary.

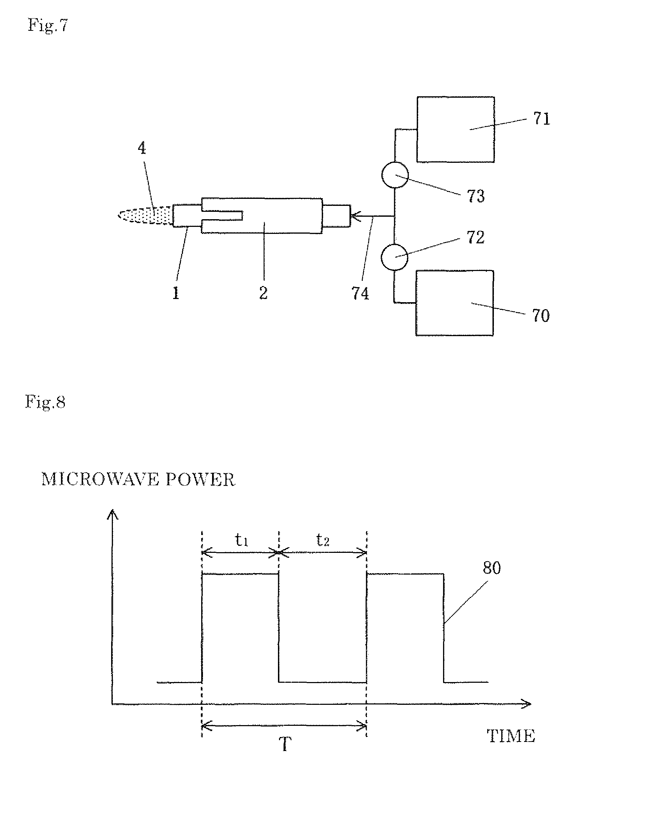

Fig 7 is a diagram illustrating another method for improving plasma ignition, according to which the difference in the energy for converting a gas to plasma depending on the type of gas is used.
70 and 7] are gas sources for supplying different types of gases, and the supply of the gases is controlled by valves 72 and 73.
Initially a gas which is put in the gas source 70 and can be easily converted to plasma is supplied to the gas flow pipe I via the valve 72 as a gas flow 74. Then, a plasma torch 4 is generated by irradiating the antenna pipe 2 with microwaves.

Next, the valve 72 is gradually closed, and at the same time, the valve 73 is opened, so that the gas supplied to the gas flow pipe 1 is switched from that from the gas source 70 to that from the gas source 71 containing oxygen. Though the gas supplied from the gas source 71 has such properties that it is difficult to convert, to plasma, plasma is already generated from the gas from the gas source 70, and therefore, it becomes easy to convert the gas from the gas source 71 to plasma. Naturally, it is also possible to supply the gases from the two gas sources 70 and 71 continuously.
Argon gas can be cited as an example of a gas that is easy to convert to plasma.

### (Pulse Drive of Plasma)

In the plasma generator according to the present invention, though it is possible to adjust the output of microwaves supplied to the antenna pipe in the plasma generating portion, so that the amount of plasma generated can be adjusted, it is also possible for plasma to be generated and remain in the gas flow pipe, unless the output of microwaves for irradiation is at a certain level or higher, in the case where the width of the slit is fixed. Thus, it becomes difficult to continuously adjust the amount of plasma generated, and therefore, in accordance with the method for generating plasma according to the present invention, this defect is compensated for with a pulse drive. In addition, the pulse drive makes it possible to prevent the gas temperature of plasma from rising, so that thermal damage of the object of sterilization due to plasma can be reduced.

Fig 8 is a graph schematically showing the change in the power of microwaves generated by the microwave generator, and a typical waveform of the drive power supplied to the microwave generator. The period T of the pulse drive consists of an ON period t1 and an OFF period (rest period) t2, and it becomes possible to continuously change the amount of plasma generated by adjusting the ratio in the duty between pulses t1/T.

Here, when the rest period t2, which becomes the period during which plasma is turned off, is too long, it becomes difficult to reignite plasma, and therefore, it is preferable for the rest period t2 to be within the average period during which plasma remains in the gas flow pipe. The average period during which plasma remains is the average value of the time after plasma is generated and before plasma disappears through contact with the surrounding gas, and varies, depending on the density of the gas and the kinetic energy of the gas converted to plasma.

### (Supply of Oxygen to Plasma)

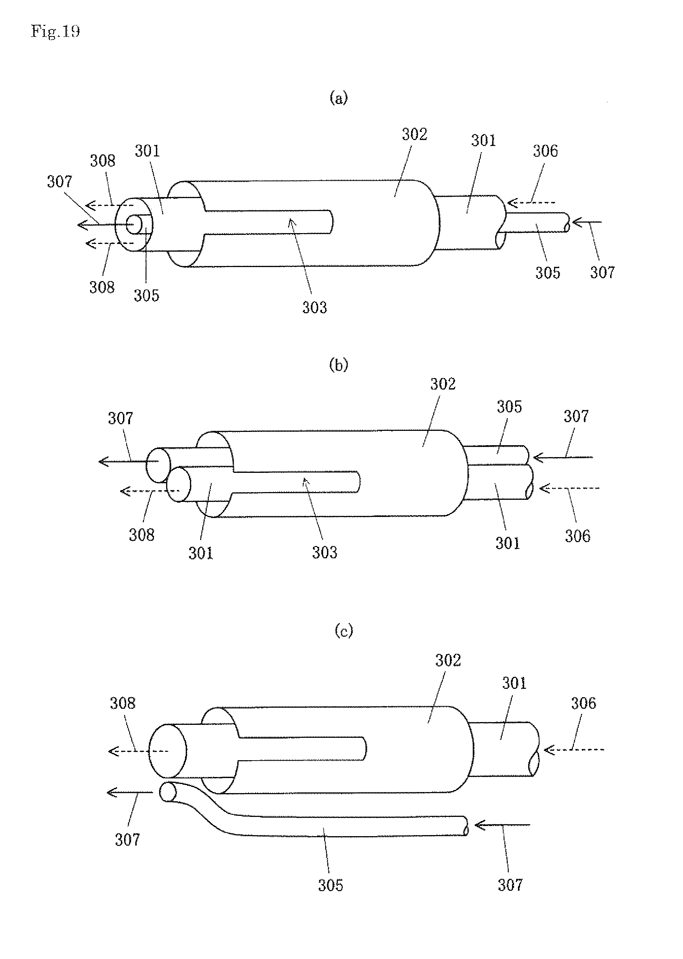

Though mainly a method for supplying oxygen to a gas to be converted to plasma using the above described plasma generating portion and plasma generator is described, it is also possible to generate active oxygen used in the sterilizer according to the present invention through contact between the gas converted to plasma and oxygen.
As shown in Fig 19(a), an oxygen gas pipe 305 for supplying oxygen into the gas flow pipe 301 is provided, so that the gas 306 supplied to the gas flow pipe 301 (may contain oxygen; a gas which can be easily converted to plasma, such as argon gas, is preferable) is converted to plasma by the antenna pipe 302 having a slit 303, and the plasma gas 308 is discharged from the gas flow pipe 301. Meanwhile, oxygen gas 307 is supplied to the oxygen gas pipe 305, and at the same time, oxygen gas 307 is discharged, so as to make contact with the plasma gas from the gas flow pipe 301. When the plasma gas 308 and the oxygen gas 307 make contact with each other, the oxygen gas is converted to active oxygen as a result of the energy of the plasma gas.

It is not necessary for the end portion of the oxygen gas pipe 305 to be in the same location as the end portion of the gas flow pipe 301, as shown in Fig 19(a), and it is also possible for the end portion of the oxygen gas pipe 305 to be located inside the gas flow pipe 301 so that the oxygen gas can be mixed with the plasma gas inside the gas flow pipe 301 in the configuration.
In addition, it is, of course, possible to generate active oxygen by supplying molecules including oxygen which can be converted to a gas instead of the oxygen gas supplied to the oxygen gas pipe 305.

Figs 19(b) and 19(c) show another example of a method for supplying oxygen to a plasma gas. Fig 19(b) shows an arrangement where a gas flow pipe 301 for supplying a gas 306 to be converted to plasma and an oxygen gas pipe 305 for supplying an oxygen gas 307 are provided side-by-side inside an antenna pipe 302, while a gas flow pipe 301 is provided in the vicinity of a slit 303 in the antenna pipe 302. In this configuration, an electrical field for excitation can be efficiently applied to the gas flow pipe 301, and thus, it becomes possible to convert the gas which flows through the gas flow pipe 301 to plasma.

Furthermore, Fig 19(c) shows an arrangement where the discharge end of an oxygen gas pipe 305 for supplying an oxygen gas is located in the vicinity of the discharge end of a gas flow pipe 301 which discharges a plasma gas 308, and thus, only a gas flow pipe 301 is provided inside the antenna pipe 302, and therefore, it becomes possible to simplify the structure for generating active oxygen.
Here, it is also possible to provide a guide pipe (not shown) in the vicinity of the discharge end of the gas flow pipe 301 and oxygen gas pipe 305 as in Figs 19(b) and 19(c), so that the guide pipe surrounds the two, so that the plasma gas 308 and the oxygen gas 307 can be efficiently mixed in the configuration.

Though in the above described examples, a separate means for supplying an oxygen gas is required, it is also possible to generate active oxygen from an oxygen gas inherent in the ambient air, for example, by irradiating ambient air with argon converted to plasma.

### (Sterilizer and Sterilizing Method)

Next, a sterilizing process using the above described plasma generator is described.
When a gas containing oxygen is converted to plasma, there are electrons, ions and active oxygen, such as oxygen radials generated through collisional dissociation from electrons within the plasma. In addition, It is possible to generate active oxygen in the same manner when a gas not including oxygen is converted to plasma, and after that the plasma gas is put in contact with a gas including oxygen, such as an oxygen gas. Such an object as a container is irradiated with this active oxygen, and thus, it becomes possible to oxidize bacteria which adhere to the surface of the object for sterilization. In addition, the plasma immediately disappears when the supply of microwaves is cut off, and at the same time, active oxygen disappears. Therefore, it becomes possible to carry out a sterilizing process extremely safely without plasma remaining. In addition, it is possible to continuously and stably generate plasma in the ambient air using the above described plasma generator, and therefore, a continuous sterilizing process can be carried out on a large amount of objects, such as containers, with the plasma used in the present invention.

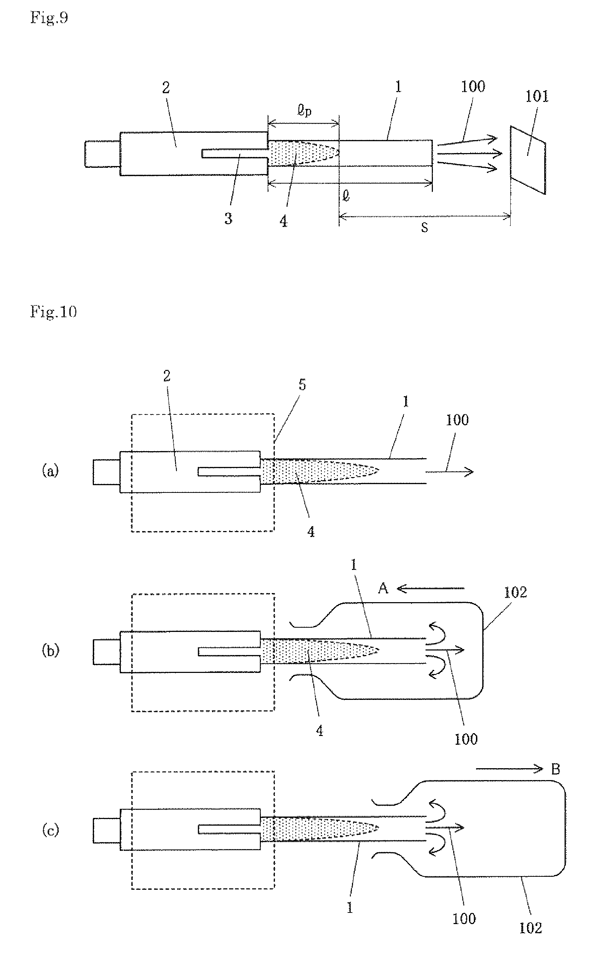

Fig 9 schematically shows the relationship between the plasma generating portion and the object 101 on which a sterilizing process is carried out.
When a gas including oxygen passes through the gas flow pipe 1 the gas is converted to plasma by the electrical field for excitation generated in the slit 3 in the antenna pipe 2. The plasma collides with the surrounding neutral gas, electrons and the like, and gradually disappears, and therefore, a plasma torch 4 as that shown in Fig 9 is generated. In front of the plasma torch 4, the gas includes a large amount of active oxygen, rather than being converted to plasma, and the gas is discharged from the end portion of the gas flow pipe 1, as indicated by arrow 100, so that an object 101 is irradiated.

In the sterilizing method according to the present invention, as shown in Fig 9, the relationship between the antenna pipe 2 and the gas flow pipe 1 is set so that the plasma torch 4 is located inside the gas flow pipe 1. Concretely, it is necessary for the length lp of the plasma torch to be greater than the length 1 of the gas flow pipe I which protrudes from the end of the antenna pipe (end portion on side where slit is created). This design prevents the plasma gas, particularly gases having a high plasma density, from making direct contact with an object, and makes it possible for the object to be protected from thermal damage caused by the plasma. It is important to prevent thermal damage caused by plasma when objects which are weak against heat, such as PET bottles are sterilized.

In addition, it is preferable to use an antenna pipe as that shown in the above Figs 17 and 18, in order to prevent the plasma torch from sticking out from the antenna pipe.
Furthermore, when the length 1 of the protruding portion of the gas flow pipe is great, the plasma and the active oxygen can be prevented from scattering the air around the gas flow pipe 1 and making contact with the surrounding air, and therefore, it becomes possible to irradiate faraway objects with active oxygen.

The distance S between the end of the plasma torch 4 and the object 101 is set to a distance which makes it possible to supply sufficient active oxygen for the sterilizing process. The amount of active oxygen depends on the gas flow of the supplied oxygen gas and the power of the applied microwaves, and is in a range from approximately several cm to several tens of cm.

### (Sterilizing Process inside Container)

Next, a method for carrying out a sterilizing process on a container which is an object of sterilization is described.
The sterilizing process is carried out on the inner surface of a container in the following procedure.

### (1) Step of igniting plasma

As shown in Fig 10(a), active oxygen 100 is released from the plasma generating portion in accordance with any of the various methods described above.

### (2) Step of inserting gas flow pipe into container

As shown in Fig 10(b), a container 102 is moved toward the gas flow pipe 1 in the direction of arrow A so that an end portion of the gas flow pipe 1 is located inside the container. How far the gas flow pipe 1 can be inserted into the container 102 depends on the length of the gas flow pipe protruding from the shielding means 5, and it is preferable for the end of the gas flow pipe to be inserted into the container so that the end reaches the vicinity of the deepest portion, as long as there is no thermal damage to the container from the plasma, in order to sterilize the inside of the container to the bottom.
Here, it is also possible in the step of inserting a gas flow pipe to move the gas flow pipe 1 so that it enters the container if necessary, instead of moving the container. In addition, it is possible to adopt various manners when the gas flow pipe is moved, for example, the antenna pipe 2 may be moved together with the gas flow pipe, or only the gas flow pipe may be moved with the antenna pipe remaining in a fixed location.

### (3) Step of filling container with active oxygen

As shown in Fig 10(b), the state where the end of the gas flow pipe is inserted intro the container so that the end reaches the vicinity of the deepest portion is held for a while, and then the container 102 is filled with a gas including active oxygen discharged from the gas flow pipe 1. The container 102 is filled with air in advance, and the end of the gas flow pipe is located in the vicinity of the deepest portion of the container, and therefore, the air is expelled from the container by the gas discharged from the gas flow pipe, and thus, the air within the container is replaced with a gas containing active oxygen.
Here, there are methods for providing a suction means for sucking air within the container in the opening of the container, and for making the outside of the container of a vacuum in order to efficiently discharge the air inside the container.

### (4) Step of withdrawing gas flow pipe

After replacing all of the air inside the container with a gas including active oxygen, as shown in Fig 10(c), the container 102 is gradually moved in the direction arrow B, and the gas flow pipe 1 is withdrawn from the vicinity of the deepest portion inside the container to the vicinity of the opening of the container. In this step of withdrawing the gas flow pipe, the gas discharged from the gas flow pipe easily makes contact with the inner surface of the container, which is located in the vicinity of the end of the gas flow pipe, and as a result, it becomes possible to supply a gas containing active oxygen throughout the entirety of the inside of the container.

Though the speed with which the gas flow pipe is inserted in the above described insertion step and the speed with which the gas flow pipe is withdrawn in the above described withdrawal step is not particularly limited, it is preferable for the speed of insertion to be high, in order to efficiently replace the air inside the container with a gas containing active oxygen, and for the speed of withdrawal to be low, because sufficient gas containing active oxygen must be supplied throughout the entirety of the inside of the container in the withdrawing step. Here, it is also possible for the speed of insertion to be as low as the speed of withdrawal, so that a sterilizing process can be carried out on the entirety of the inner surface of the container in the insertion step in the configuration.
In addition, it is also possible to repeat the steps from the above descried insertion step to the withdrawal step a number of times for the same container if necessary.

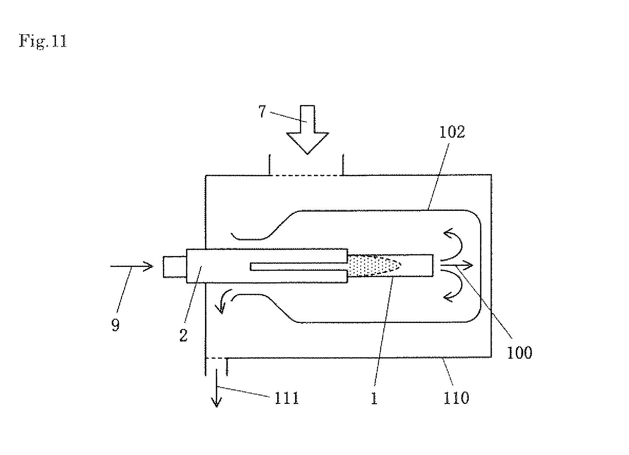

Next, another method for carrying out a sterilizing process on the inside of a container is described.
As shown in Fig 11, a container 102 contains part of a gas flow pipe 1 and an antenna pipe 2, and a shielding means 110 surrounding the container 102 is provided. Thus, the antenna pipe 2 is irradiated with microwaves 7 while a gas is discharged into the container from the gas flow pipe 1, so that it becomes possible to supply a gas 100 containing active oxygen into the container.
As described above, the end of the antenna pipe 2 can be contained within the container, and therefore, it becomes possible to supply active oxygen stably in the deepest portion of the container, even in the case where the container is deep. Here, it is necessary to irradiate the antenna pipe 2 with microwaves, and therefore, this method can be used only for containers 102 made of a non-conductive material which can transmit microwaves.

In the sterilizer shown in Fig 11, a discharge opening is created in a portion of the above described shielding means 110 in order to discharge the gas inside the shielding means to the outside, and it is preferable to supply a gas into the gas flow pipe while discharging the gas 111 through the discharge opening in the configuration. As a result, it becomes possible to first discharge the air inside the container through the discharge opening, before a gas containing active oxygen is supplied, so that the degree of vacuum in the container becomes higher, and to replace the air inside the container with active oxygen rapidly by subsequently supplying a gas containing active oxygen. In addition, it is also possible to keep the air pressure at the time of plasma ignition in the plasma generating portion lower than the ambient pressure, and thus, it is also possible to improve plasma ignition.

Furthermore, it is, of course, possible to secure sufficient space inside the shielding means 110 for the container 102 to be movable relative to the gas flow pipe 1 inside the shielding means 110, as shown in Fig 10.

### (Sterilizing process outside container)

Next, a sterilizing process outside the container is described.
Fig 12 shows an example of a0 method for carrying out a sterilizing process on the outside of a container: Fig 12(a) is a perspective diagram showing a sterilizer, and Fig 12(b) is a cross sectional diagram along arrow X in Fig 12(a).
In the sterilizer in Fig 12, a gas discharged through the gas flow pipe 1 is blown against the outer surface of the container 102, and a means for rotating the container 102 around an axis which is approximately perpendicular to the axis of the gas flow pipe 1 is provided. Though two rollers 120 are illustrated as the rotating means, the present invention is not limited to this, and any means can be adopted, as long as it is possible to rotate the container 102 relative to the gas flow pipe 1.

As described above, a gas containing active oxygen can be supplied to the outer surface of the container from the gas flow pipe 1 while rotating the container 102, and therefore, it becomes possible to carry out a sterilizing process over the entire outer surface of the container.
Furthermore, the gas discharged from the gas flow pipe 1 efficiently makes contact with the container 102, and therefore, it is also possible to provide a hood 121 in the vicinity of the discharge end of the gas flow pipe, so that the gas discharged from the gas flow pipe 1 can be guided to the outer surface of the container 102 in the configuration.

In addition, as another method for carrying out a sterilizing process on the outside of a container, as shown in Fig 13, a space for temporarily holding a gas discharged from the gas flow pipe 1 is created, so that the container 102 can be conveyed through the space.
As the space for holding a gas, as shown in Fig 13, a tunnel 130 can be used. A number of plasma generating portions are connected to the tunnel 130, so that the tunnel 130 is filled with the gas discharged from the gas flow pipe 1 in the configuration. The length of the tunnel is set in accordance with the speed of conveyance of the container 102, and the length is sufficient for the container 102 to make contact with the gas containing active oxygen with which the tunnel is filled when passing through the tunnel, and thus, a sterilizing process is carried out.

As shown in Fig 13, containers 102 are continuously conveyed into the tunnel 130 in the direction of the arrow 131 and conveyed out in the direction of the arrow 132. Thus, it becomes possible to carry out a sterilizing process while continuously conveying containers, and therefore the sterilizing process can be carried out extremely efficiently. In addition, the containers 102 can be rotated around their center axis within the tunnel if necessary, and thus, it is also possible to irradiate the outer surface of the containers with the gas containing active oxygen uniformly. Furthermore, it is also possible to provide plasma generating portions on the side of or beneath the tunnel 130 instead of above the tunnel 130, as shown in Fig 13.

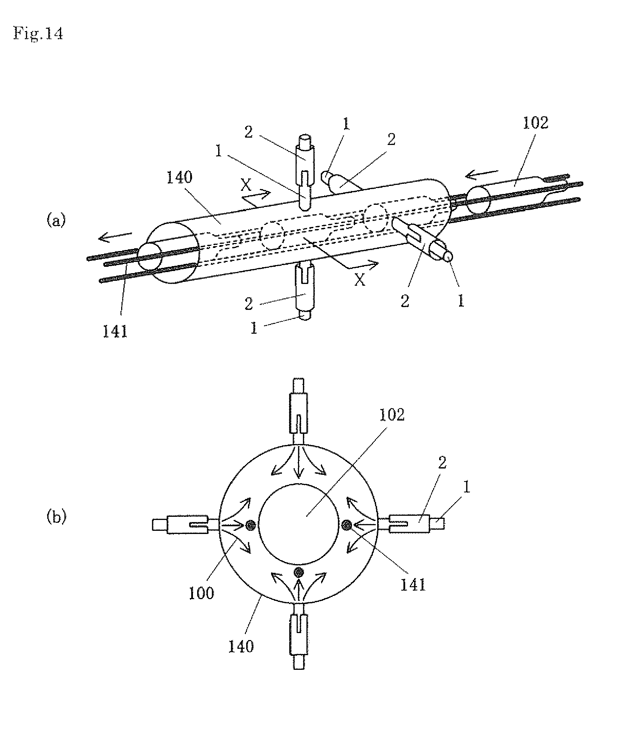

Fig 14 shows an example where the space for holding a gas is modified so as to be in approximately cylindrical form. Plasma generating portions are provided around the tunnel 140, and the end of each gas flow pipe 1 is connected to the inside of the tunnel 140. Guide means 141 for conveying containers 102 pass through the tunnel 140. When the containers 102 move along the guide means 141, a sterilizing process is carried out on the outer surface of the containers 102 with the gas with which the tunnel 140 is filled.

Fig 14(b) is a cross sectional diagram along the arrow X in Fig 14(a), and as shown in Fig 14(b), a number of plasma generating portions are provided around the container 102 so that the gas 100 containing active oxygen can be supplied over the entire outer surface of the container 102.
The container 102 works to push out the space inside the tunnel 140 when the container 102 passes through the tunnel 140. Therefore, it is preferable to supply a greater amount of gas from the gas flow pipe 1 than pushed out by the container 102, and there is always fresh gas containing active oxygen in the space within the tunnel, due to the effects of expelling gas.

### (Sterilizing process on lid of container)

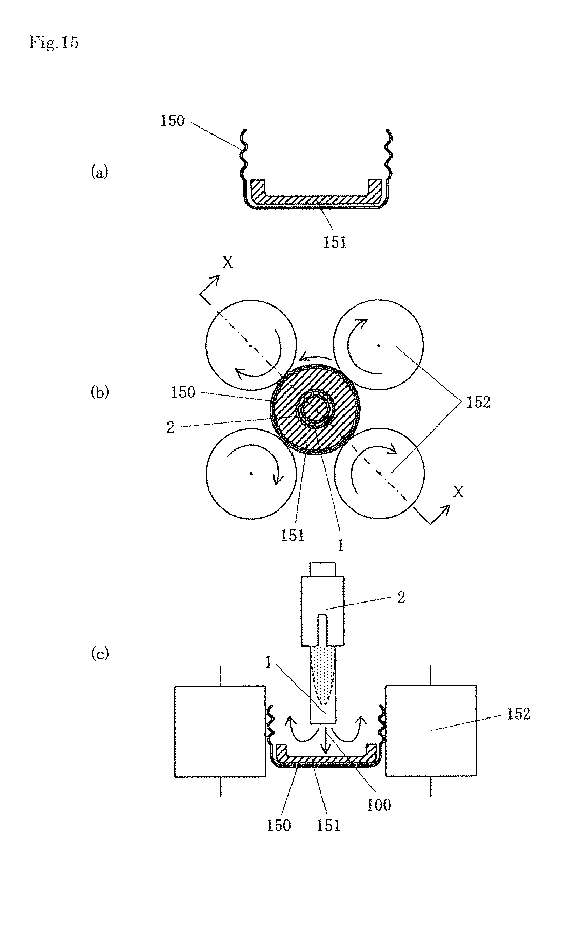

In some cases, the lid of a container is provided with a sealing member 151 made of rubber or plastic inside the lid 150, as shown in Fig 15(a). In the case were such a sealing member 151 is provided, active oxygen does not get into the space between the sealing member 151 and the lid 150, simply when a gas containing active oxygen is blown against the inside of the lid 150, and thus, there is a risk that the sterilizing process may be insufficient when there are germs in the space.

In order to solve this problem, as shown in Figs 15(b) and 15(c), the lid 150 is irradiated with active oxygen while being rotated. Fig 15(c) is a cross sectional diagram along the arrow X in Fig 15(b).
Rollers 152 are provided around the lid 150, so that the lid can be rotated in the configuration. The rotating means is not limited to rollers, and any means which makes it possible to rotate the lid may be used.

Though when the lid 150 rotates, the air in the space between the lid 150 and the sealing member 151 is discharged from the lid 150 due to centrifugal force, it becomes possible to carry out a sterilizing process for killing bacteria in the space, so that the gas containing active oxygen with which the lid is irradiated gets into the space when the speed of rotation is reduced or rotation is stopped. As described above, a gas containing active oxygen repeatedly enters the space, so that a sterilizing process is carried out by changing the speed, direction and the like for rotating the lid 150 a number of times.

Fig 16 illustrates another method for carrying out a sterilizing process on lids 161.
Lids 161 have a sealing member, not shown, inside, as in Fig 15(a). Lids are provided on shelves 162 provided inside a vacuum container 160, and the air inside the vacuum container is discharged 163 using a vacuum pump, so that the pressure inside the vacuum container lowers. During the process for reducing the pressure, air remaining in the space between the lid and the sealing member is discharged, and after that, a gas containing active oxygen is supplied into the vacuum container 160 from the plasma generating portion, and thus, it becomes possible for active oxygen to enter the space.

The flow amount, of gas 163 expelled from the vacuum container relative to the flow amount, of gas 100 containing active oxygen is changed so that it becomes possible for the gas 100 to enter into the above described space repeatedly. That is to say, in the case where the flow amount of the gas 163 is greater than that of the gas 100, the gas is discharged through the above described space, while in the case where the flow amount of the gas 163 is smaller than that of the gas 100, the gas 100 enters the above described space.

### Example 1

The results of experiments using the sterilizer according to the present invention are described below.
In the plasma generating portion shown in Fig 18(b), a quartz pipe (inner diameter 20 mm, outer diameter: 22 mm) was used as the gas flow pipe, and a pipe made of aluminum (inner diameter: 26 mm, outer diameter: 28 mm) was used as the antenna pipe. One slit having a width D of 5 mm and a length L or 60 mm was created in the antenna pipe.

The plasma generating portion made up of the antenna pipe and the gas flow pipe was provided inside a chamber having an inner diameter of 160 mm and a length 1500 mm, which became a shielding means.
The pressure inside the gas flow pipe was reduced to 10² Pa, and at the same time, a gas flow of oxygen of 0.3 (l/min) and a gas flow of argon of 3.0 (l/min) were introduced into the gas flow pipe, and furthermore, microwaves, (frequency 2.45 GHz) were introduced into the chamber with a power for emitting microwaves of 1000 W.

After plasma ignition, the inside of the gas flow pipe was opened so that the inside became of the ambient pressure (10⁵ Pa) and the emission spectrum of oxygen atoms irradiated through the slit of the antenna pipe was observed, and it was found that the intensity in the spectrum at 777 nm was 2.9 (a.u.). When the gas supplied to the above described gas flow pipe was only argon gas, the intensity in the same spectrum was 1.0 (a.u.), and therefore, it could be confirmed that when the above described oxygen gas was included, the oxygen gas dissociated stably, so that oxygen radicals were generated.

Next, bacteria taken from rotten pork were put on the surface of one test piece of a PET material, and the test piece was cut in half, and thus, two test pieces having a length of 40 mm × a width of 25 mm were prepared. One of the test pieces was placed in a location 3 cm away from the end portion of the antenna pipe (lp + S), as shown in Fig 9 and irradiated with a gas containing active oxygen for 3 seconds.

After the sterilizing process, the bacteria on the test piece were cultivated for 24 hours at 35 °C, and the number of colonies was counted. In order to compare the effects of sterilization, the bacteria on the other test piece were cultivated in the same manner without irradiation with a gas containing object oxygen, and the number of colonies was counted. The results after the above described experiments were carried out twice are shown in Table 1.

**[Table 1]**

| | not irradiated with active oxygen | irradiated with active oxygen |
|---|---|---|
| first time | 520 | 0 |
| second time | 1044 | 0 |

It can be confirmed from the results in Table 1 that 100 % of bacteria were killed in the case where the test piece was processed in the sterilizer according to the present invention. It can be understood from this that the sterilize and the sterilizing method using the sterilizer according to the present invention have rapid and highly excellent sterilizing effects.

### Example 2

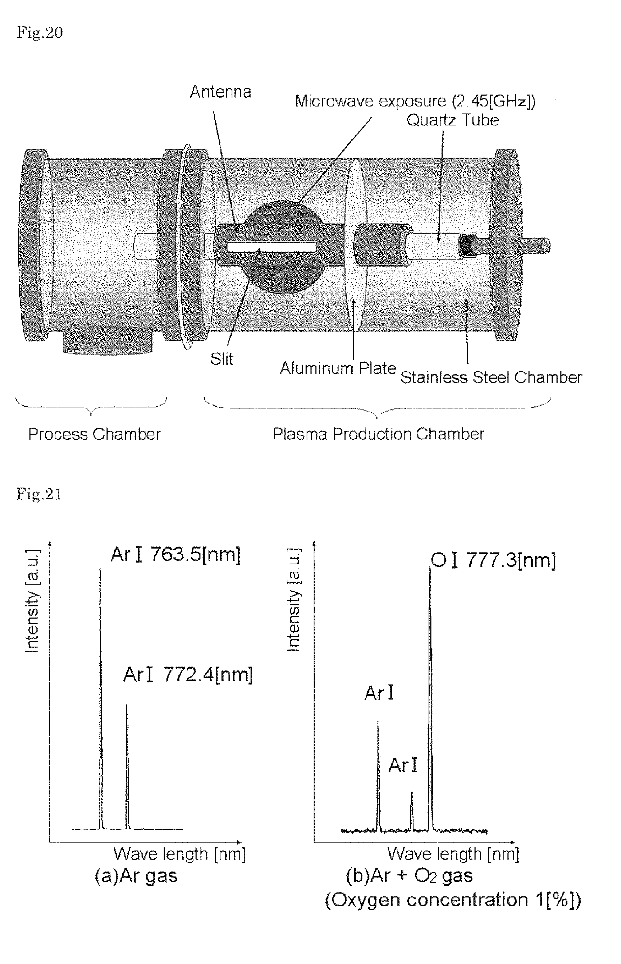

Next, an experiment, was conducted using the plasma generator shown in Fig 20.
The plasma generator was divided into two main parts: one was a plasma production chamber, and the other a process chamber. It becomes possible to irradiate various objects with radicals by providing a process chamber. The inside of the plasma production chamber was partitioned with a shield plate made of aluminum, and a crystal pipe (inner diameter: 10 mm, outer diameter: 13 mm) passed along the center axis of the shield plate so as to extend to the process chamber. Furthermore, the crystal pipe is coated with an antenna made of aluminum in cylindrical form, as shown in Fig 18(b), and two slits having a length of 60 mm (width of 5 mm) corresponding to the half wavelength of the microwaves were created in the antenna at symmetrical points along the periphery of the antenna pipe, and one of these was oriented toward an opening through which microwaves enter.

In accordance with one example of the method for generating plasma, the air inside the quartz pipe and the process chamber was discharged using a rotary pump, and after that, an argon gas was fed through the quartz pipe with the gas pressure kept at 100 Pa to 200 Pa, and after that, the quartz pipe was irradiated with microwaves through a waveguide (frequency: 2.45 GHz), and thus, argon gas plasma was generated. After that, when the switching lever of the rotary pump was operated so as to raise the gas pressure to the ambient pressure, nonequilibrium plasma was kept under the ambient pressure (state of plasma in which electron temperature is as high as several tens of thousands of degrees or higher, but ion temperature or gas temperature is several tens of degrees to several hundreds of degrees). The plasma generated in the plasma generating portion was blown out into the plasma process chamber together with the gas flow.

### (Observation of emission spectrum of plasma)

The emission spectra in the case where plasma was generated and remained in the gas flow pipe and only all argon gas was used (Fig 21(a)), as well as in the case where a mixture gas of argon and oxygen was used (Fig 21(b)), is shown in Fig 21. The conditions for measurement are such that the amount of gas flow of argon was 6.0 [l/min] in both cases, and the amount of gas flow of oxygen was 0.07 [l/min] (mixture ratio: approximately 1 %) in Fig 18(b). In addition, the power for emitting microwaves was 600 W.

in Fig 21(a), spectrum lines (ArI) were observed for argon atoms at a wavelength of 763.5 nm and 772.4 nm, while in Fig 21(b), a considerably intense spectrum line (OI) was observed for oxygen atoms at a wavelength of 777.3 nm, in addition to the spectrum lines in Fig 21(a). In the present sterilizer, intense light emission from oxygen atoms was observed despite the oxygen mixture ratio being approximately 1 %, and this is considered to be because the oxygen molecules dissociated efficiently, and thus there were many oxygen atoms (oxygen radicals) in the plasma.

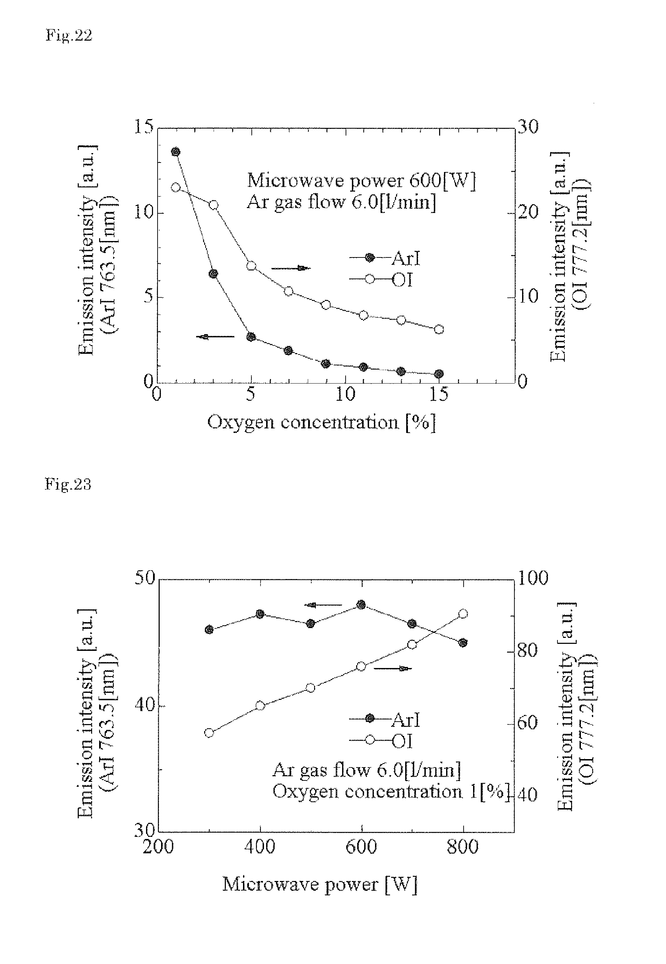

Next, the dependency of the intensity of light emission on the oxygen gas mixture ratio and the power for emitting microwaves was examined for both ArI (763.5 nm) and OI (777.3 nm). The results are shown in Fig 22 (dependency on oxygen gas mixture ratio) and Fig 23 (dependency on power for emitting microwaves).

### (Dependency on oxygen Gas Mixture Ratio)

In order to examine the dependency on the oxygen gas mixture ratio, the oxygen gas mixture ratio was changed within a range of 1 % to 15 % with a power for emitting microwaves of 600 W and a flow of argon gas of 6.0 [l/min], and only the flow of oxygen gas (oxygen content) was changed. It can be seen from Fig 22 that the intensity of emitted light for both argon and oxygen atoms suddenly decreased as the concentration of oxygen increased. It was actually observed by the eye that the intensity of light emitted from the plasma as a whole decreased when an oxygen gas was mixed in. This is considered to be because the energy of microwaves was used for the dissociation of oxygen molecules, in addition to ionization and excitation, due to the presence of oxygen in the form of molecules.

### (Dependency on Power for Emitting Microwaves)

Next, in order to examine the dependency on the power for emitting microwaves in such a state that plasma is generated, the power for emitting microwaves was changed within a range of 300 W to 800 W with a flow of argon gas of 6.0 [l/min] and a flow of oxygen gas of 0.07 [l/min] (oxygen mixture ratio: approximately 1 %). It can be seen from Fig 23 that the intensity of light emitted from argon atoms did not change much, while the intensity of light emitted from oxygen atoms increased together with the power for emitting microwaves when the power for emitting microwaves was increased. This is considered to be because the energy required for dissociation of oxygen molecules is considerably lower than the energy required for dissociation of argon atoms, and therefore, the extra power for emitting microwaves was consumed by the dissociation of oxygen molecules instead of argon atoms.

### (Measurement of Distance Traveled by Active Oxygen)

In order to examine how far active oxygen, particularly oxygen radicals released from the crystal pipe, traveled, a chemical indicator strip which reacts with oxygen radicals (product name: STERRAD Chemical Indicator Strip, product number: REF 14100, made by ASP Company) was placed at a distance of 6 cm to 10 cm from the end of the quartz pipe in the direction in which the quarts pipe extend, and the reaction of the indicator was examined. The distance between the end of the quartz pipe and the end of the antenna pipe was 20 mm, and the distance between the end of the antenna pipe and the end of the slit (end on side from which gas discharged) was 3 mm. In addition, the flow of argon gas was constant at 6.0 [l/min], and the flow of oxygen gas was adjusted so that the oxygen gas mixture ratio varied within a range of 0 % to 2 %. In addition, the power for emitting microwaves was 600 W.

The results of measurement are shown in Table 2. In the case where the color of the indicator changed from purple to yellow, it was determined that oxygen radicals were present, and therefore, the evaluation was ○, and little change or no change at all was evaluated as ×.

**[Table 2]**

| oxygen gas mixture ratio [%] | 10[cm] | 9[cm) | 8[cm] | 7[cm] | 6[km] |
|---|---|---|---|---|---|
| 0 | × | × | × | × | ○ |
| 1 | × | ○ | ○ | ○ | ○ |
| 1.3 | × | ○ | ○ | ○ | ○ |
| 1.5 | × | ○ | ○ | ○ | ○ |
| 1.6 | × | ○ | ○ | ○ | ○ |
| 1.8 | × | ○ | ○ | ○ | ○ |
| 2.0 | × | ○ | ○ | ○ | ○ |

It was found from the results shown in Table 2 that oxygen radicals were present up to 9 cm from the end of the quartz pipe, irrespectively of the oxygen gas mixture ratio. Thus, it was found that oxygen radicals were present over a long distance despite the pressure being ambient.
In addition, it was found that oxygen radicals were present up to 6 cm from the end of the quartz pipe in the case of only argon gas and no oxygen gas. This is considered to be because argon atoms in a semi stable state which were excited in the antenna portion and had a long life dissociated oxygen molecules included in the air within the process chamber, so that oxygen atoms (radicals) were generated.

### (measurement of gas temperature)

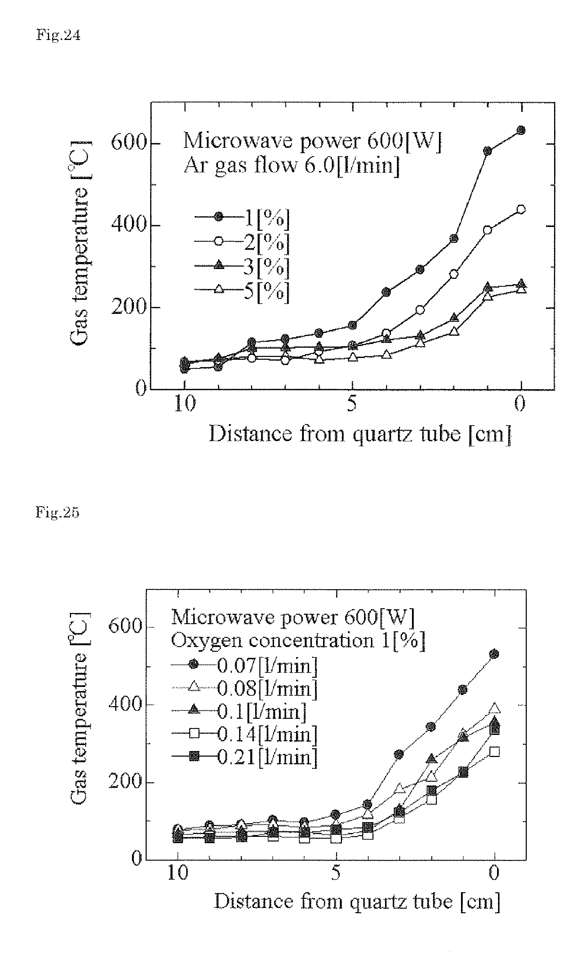

In order to find out the temperature distribution throughout the process chamber, thermocouples were placed at a distance of 0 cm to 10 cm from the end of the quartz pipe in the direction in which the quartz pipe extends, and the gas temperature was measures. The results of measurement are shown in Fig 24. Here, the flow of the argon gas was constant at. 6.0 [l/min] and the flow of oxygen gas was adjusted so that the oxygen gas mixture ratio varied within a range of I % to 5 %. In addition, the power for emitting microwaves was 600 W.

It can be seen from Fig 24 that the gas temperature rapidly decreased as the distance from the discharge end increased for all gas mixture ratios. This is considered to be because gas particles of a high temperature generated in the antenna portion collide with other particles when they move within the process chamber, and thus lost energy rapidly. In addition, as the oxygen mixture ratio increased, the gas temperature tended to lower as a whole. This is considered to be because the energy given electrons by the microwaves was consumed for the dissociation of oxygen molecules as the number of oxygen molecules increased, and thus, application of heat to the gas particles by electrons was prevented, as in the case of the dependency of the intensity of light emission on the oxygen mixture ratio shown in Fig 22. In addition, it can be seen from Fig 24 that the gas temperature was as low as 80 °C or lower, irrespectively of the oxygen mixture ratio, at a point 9 cm from the end of the quartz pipe.

Next, the change in the distribution of gas temperature was found for the case where the oxygen gas mixture ratio was fixed at 1 % and the gas flow was changed. The gas flow of oxygen was changed within a range of 0.07 [l/min] to 0.21 [l/min], and the flow, of argon was adjusted together with this, so that the mixture ratio of oxygen gas remained at 1 (%. The power for emitting microwaves was 600 W. The results of measurement are shown in Fig 25.

It can be seen from Fig 25 that the gas temperature towered when the gas flow increased. When the gas flow increased, the speed of gas flow increased, and in this case, the time required for the gas to pass through the region where plasma is generated (antenna portion) was shorter. That is to say, the number of collisions with electrons having high energy decreased, and therefore, the gas temperature is considered to have lowered when the gas flow increased.

### (Evaluation of Sterilizing Effects in Accordance with Colony Counting Method)

The colony counting method is a method for collecting bacteria from the surface of an object after a sterilizing process and finding the sterilizing ratio from the number of colonies (groups of bacteria) after cultivating the bacteria in a culture medium. In the following, the procedure for the colony counting method used in the present experiment is explained.

(1) Bacillus atrophaeus, which is an indicator bacterium, (KWIK-STIKTM 10PK 0953S, MiercoBiologics Corporation), were applied uniformly on an aluminum plate (2 cm × 2 cm). Here, the Bacillus atrophaeus were sporulative bacteria resistant to a temperature of approximately 80 C. They were used as indicator bacteria for heat sterilization and gas sterilization.
(2) Next, half of the aluminum plate was wiped with a swab, and the swab was dipped in a culture solution.
(3) The aluminum plate was installed in a plasma generator and a sterilizing process was carried out.
(4) After the sterilizing process, the aluminum plate was taken out and the remaining half was wiped with a swab as in the above, and the swab was put in another culture solution.

(5) The same amount of the two culture solutions was dropped onto different culture media (Sanita kun, Chisso Corporation). The bacteria in the culture media were cultivated for 40 hours in an incubator with the temperature set to 35 °C, which is the optimum temperature for Bacillus atrophaeus to grow. When bacteria are present, colonies appear in the culture medium in accordance with the number.
(6) The sterilizing ratio was found from the number of colonies before the sterilizing process (the above (2)) and the number of colonies after the sterilizing process (the above (3)).

The conditions for the sterilizing process using plasma in the above (3) were such that the power for emitting microwaves was 600 W, the flow of argon gas was 6.0 [l/min], the time for processing was 3 seconds, the area processed (distance from end of quartz pipe in direction in which quartz pipe extends) was 9 cm, and the oxygen mixture ratio was changed within a range of 0 % to 2 %. The results of measurement are shown in Table 3.

**[Table 3]**

| Ar gas flow [ℓ/min] | Oxygen concentration [%] | Colony count (Before treatment) | Colony count (After treatment) | Sterilization rate [%] |
|---|---|---|---|---|
| 6 | 0 | 2325 | 25 | 98.9 |
| 6 | 1 | 2000 | 2 | 99.9 |
| 6 | 2 | 2050 | 14 | 99.3 |

It can be seen from Table 3 that a high sterilizing ratio was achieved in all cases, despite the time for processing being as short as 3 seconds. Though generally, heat, ultraviolet, rays and oxygen radicals are cited as causes for bacteria dying, bacteria of the Bacillus genus are thermophilic, and thus not killed at a temperature of 80 [°C]. In addition, judging from the results of measurement of the gas temperature, the gas temperature in the area where the sterilizing process was carried out was approximately 55 °C in the case where the oxygen gas mixture ratio was 1 %, and approximately 70 °C in the case where the mixture ratio was 2 %. Furthermore, no ultraviolet rays having a wavelength of 200 nm to 280 nm and a high efficiency for sterilization could be observed. In addition, the presence of oxygen radicals was confirmed in the area where the sterilizing process was carried out from Table 2, and therefore, the sterilizing effects for these bacteria were assumed to be the work of oxygen radicals.
In addition, sterilization was achieved in the case where the oxygen mixture ratio was 0 %, as shown in Table 3, and this was possibly because semi-stable argon atoms having a high internal energy reacted with each other and with oxygen in the air so as to work as sterilizers.

### (Evaluation of Sterilizing Effects Using Biological Indicator)

Test paper (biological indicator); that is, filter paper on which there was a large amount of bacteria (1 × 10⁶), was used to evaluate the sterilization. The biological indicator used (made by Raven Company) was a piece of filter paper of 6.4 mm × 38.1 mm on which there were 106 spores of Bacillus stearothermophilus.

In the experiments, this biological indicator was cut into six pieces for use. In addition, the bacteria used were sporulative and resistant to a temperature of 120 [C]. They are used as indicator bacteria for heat sterilization.

In the hollowing, the procedure for sterilization using a biological indicator is explained.
(1) A cut biological indicator was put in the process chamber and plasma sterilization was carried out.
(2) The biological indicator was taken out and put in a test tube in which there was a culture solution (trypsin soy broth).
(3) The test tube was put in an incubator and the bacteria cultivated for 72 hours at 60 °C. In the case where the color of the culture solution within the test tube remained purple, hence did not change, after 72 hours, it was determined that no bacteria were present (evaluation ○ in Table 4), and in the case where the color changed to yellow, it was determined that bacteria had survived (evaluation X in Table 4).

The conditions for the sterilizing process using plasma were such that the power for emitting microwaves was 600 W, the flow of argon gas was 6.0 [l/min], and the irradiated area (distance from end of quartz pipe in direction in which quart pipe extends) was 9 cm. When the sterilizing process was carried out, one side of the test paper was first directed toward the discharge end for processing, and likewise, the other side of the test paper was directed toward the discharge end for processing. The two surfaces were processed for the same time. The time for processing in Table 4 indicates the total time for processing the two sides.

**[Table 4]**

| Oxygen concentration [%] | Exposure time [s] | | | | |
|---|---|---|---|---|---|
| | 10(5 × 2) [s] | 20(10 × 2) [s] | 30(15 × 2) [s] | 40(20 × 2) [s] | 50(25 × 2) [s] |
| 1 | × | × | × | × | ○ |
| 2 | × | × | × | × | ○ |
| 3 | × | × | × | × | ○ |
| 5 | × | × | × | × | × |

The gas temperature in the processed area (approximately 55 °C to 75 °C) was sufficiently lower than the temperature for killing bacteria (120 or higher), and almost no ultraviolet rays were observed, and therefore, sterilization was considered to be the work of oxygen radicals in the present experiment. It can be seen from Table 4 that a processing time of 50 seconds was required to kill the bacteria for an oxygen mixture ratio of 1 % to 3 %. In addition, the bacteria could not be killed during the processing time for an oxygen mixture ratio of 5 %. This is assumed to be because the number of excited oxygen radicals (having internal energy) decreased as the oxygen mixture ratio increased.

For reference, a sterilizing process was carried out on only one side of the test paper in a separate experiment under such conditions that the power for emitting microwaves was 600 W, the flow of argon gas was 8.6 [l/min] and the oxygen mixture ratio was I %. The processed area was changed within a range of 6 cm to 9 cm, and it was confirmed that sterilization was successful in 25 seconds or longer in all areas. Here, the gas temperature in the processed area was (18.8 °C at 9 cm, 72.9 °C at 8 cm, 74.5 °C at 7 cm and 72.2 °C at 6 cm.

It can be understood from the above described results the 6D value of Bacillus atrophaeus (processing time required for the number of surviving strains of bacteria to become 10⁻⁶) was approximately 9 seconds, and the value for Bacillus stearothermophilus was approximately 30 seconds in the sterilizer according to the present invention. This shows that the sterilizing process could be in an extremely short period of time, in comparison with the three minutes required in high pressure steam sterilization (autoclave method), which is widely used at present.

The sterilizer and the sterilizing method according to the present invention are not limited to those above, and the above various technologies, for example those relating to the plasma generator, the auxiliary igniting means and the pulse drive for plasma, can, of course, be used in the above described sterilizer and sterilizing method.
Here, though mainly examples where an oxygen gas is used are described for the above sterilizer and sterilizing method, it is also possible to use OH radicals which are more potent as oxidants instead of oxygen radicals generated from an oxygen gas. In the case where OH radicals are used, it is possible to shorten the time for the sterilizing process. Here, in order to generate OH radicals, a vaporized water gas of which the cost is as low as the material for venerating plasma can be used.

### Industrial Applicability

As described above, the present invention can provide a sterilizer for efficiently carrying out a sterilizing process on such an object as a container using active oxygen generated using plasma, and a sterilizing method using the same. In addition, the present invention makes it possible to stably generate plasma under the ambient pressure, so that a sterilizing process can be carried out on a large amount of objects to be sterilized, such as containers.

## Claims

1. A sterilizer for putting a gas which includes at least active oxygen generated by converting an oxygen gas to plasma or active oxygen generated by converting a gas other than oxygen to plasma and putting the plasma in contact with an oxygen gas in contact with an object so that bacteria which adhere to the object can be killed, **characterized in that** the sterilizer comprises:
a non-conductive gas flow pipe through which a gas to be converted to plasma is introduced and discharged into the air; and
a conductive antenna pipe surrounding the gas flow pipe, wherein
a slit of a predetermined length is created in the antenna pipe along the pipe axis of the gas flow pipe, and
the antenna pipe is irradiated with microwaves to that the gas flowing through the gas flow pipe is converted to plasma.

2. The sterilizer according to Claim 1, **characterized in that** the slit is open on the side of the gas flow pipe from which the gas is discharged

3. The sterilizer according to Claim 1, **characterized in that** the slit is created inside the antenna pipe.

4. The sterilizer according to any of Claims 1 to 3, **characterized in that** the end portion of the antenna pipe on the side from which the gas is discharged from the gas flow pipe is bent toward the gas flow pipe.

5. The sterilizer according to any of Claims 1 to 4, **characterized in that** the length of the slit is set to a multiple of the half wavelength of microwaves for irradiation in integers.

6. The sterilizer according to any of Claims 1 to 5, **characterized in that** the gas flow pipe protrudes from the end portion of the antenna pipe on the downstream side in the direction of the gas flow, and the length of the protruding portion is greater than the length of the plasma torch.

7. The sterilizer according to any of Claims 1 to 6, **characterized in that** the gas flow pipe and the antenna pipe can be moved relative to each other in the configuration.

8. The sterilizer according to any of Claims 1 to 7, **characterized in that** an oxygen gas pipe for introducing an oxygen gas is provided inside the gas flow pipe or around the gas flow pipe, so that plasma generated inside the gas flow pipe and the oxygen gas introduced through the oxygen gas pipe make contact.

9. The sterilizer according to any of Claims 1 to 8, **characterized in that** the object of sterilization is a container or a lid of a container.

10. The sterilizer according to Claim 9, **characterized by** comprising a moving/rotating means for moving or rotating the object relative to the gas flow pipe.

11. A sterilizing method using the sterilizer according to Claim 10, **characterized in that** the objects of sterilization is a container, comprising: the insertion step of inserting an end of the gas flow pipe so that the end reaches the vicinity of the deepest portion inside the container; the filling step of filling the container with the gas discharged from the gas flow pipe in the state after the insertion step; and the drawing step of drawing the gas flow pipe from the vicinity of the deepest portion inside the container to in the vicinity of the opening of the container after the filling step.

12. The sterilizing method according to Claim 11, **characterized in that** the insertion step and the drawing step are carried out by moving the gas flow pipe and the container relative to each other along the pipe axis of the gas flow pipe.

13. The sterilizing method according to Claim 12, **characterized in that** the speed of the relative movement between the gas flow pipe and the container is slower in the drawing step than in the insertion step.

14. A sterilizing method using the sterilizer according to any of Claims 1 to 8, **characterized in that** the object of sterilization is a container, the container contains part of the gas flow pipe and the antenna pipe, and a shielding means is provided so as to surround the container, and thus, the antenna pipe can be irradiated with microwaves while the gas is being discharged into the container from the gas flow pipe.

15. The sterilizing method according to Claim 14, **characterized in that** a discharge hole for discharging the gas from inside the shielding means is created in a portion of the shielding means, so that a gas can be supplied to the gas flow pipe while air is discharged through the discharge hole.

16. A sterilizing method using the sterilizer according to any of Claims 1 to 8, **characterized in that** the object of sterilization is a container, and a space for temporarily storing the gas discharged from the gas flow pipe is provided, so that the container can be conveyed through the space.

17. The sterilizing method according to Claim 16, **characterized in that** the space is a tunnel, and the discharge end of the gas flow pipe connects to the space.

18. A sterilizing method using the sterilizer according to any of Claims I to 8, **characterized in that** the object of sterilization is a container, a gas discharged from the gas flow pipe is blown against the outer surface of the container, and the container is rotated around the center axis in a direction approximately perpendicular to the pipe axis of the gas flow pipe.

19. The sterilizing method according to Claim 18, **characterized in that** a hood for putting the gas discharged from the gas flow pipe in contact with the container is provided in the vicinity of the discharge end of the gas flow pipe.

20. A sterilizing method using the sterilizer according to any of Claims 1 to 8, **characterized in that** the object of sterilization is a lid of a container, the discharge end of the gas flow pipe is directed toward the inside of the lid so that a gas can be supplied to the lid from the gas flow pipe, and the lid can be rotated.

21. The sterilizing method according to Claim 20, **characterized in that** the speed of rotation and the direction of rotation of the lid is changed over time.

22. A sterilizing method using the sterilizer according to any of Claims 1 to 8, **characterized in that** the object of sterilization is a lid of a container, and the lid is put in a vacuum container, so that a gas can be supplied into the vacuum container from the discharge end of the gas flow pipe while air is discharged from the vacuum container.

23. A sterilizing method using the sterilizer according to any of Claims 1 to 8, **characterized by**
the plasma igniting step of supplying a gas which is easier to convert to plasma than oxygen gas to the gas flow pipe and igniting the gas into plasma through irradiation with microwaves, and
supplying an oxygen gas together with said gas after the plasma igniting step so that the oxygen gas is converted to plasma.

24. The sterilizing method according to Claim 23, **characterized in that** the gas supplied in the plasma igniting step is an argon gas.
